# EUROPEAN PATENT APPLICATION

(11) **EP 4 273 167 A1**
(43) Date of publication of application: **08.11.2023**
(21) Application number: 21914526.5
(22) Date of filing: 29.12.2021
(51) Int. Cl.: C07K 16/28, C07K 16/30, C12N 15/13, C12N 15/85, C12N 5/10, G01N 33/577, G01N 33/574, A61K 39/395, A61P 35/00

(54) **ANTI-CLDN18.2 ANTIBODY, AND PREPARATION METHOD THEREFOR AND USE THEREOF**

(30) Priority: 30.12.2020 WO PCT/CN2020/141761
(71) Applicant: Bio-Thera Solutions, Ltd., Guangzhou, Guangdong 510530 (CN)
(72) Inventor: QIN, Chao, Guangzhou, Guangdong 510530 (CN); XIAO, Cuizhen, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: dompatent von Kreisler Selting Werner - Partnerschaft von Patent- und Rechtsanwälten mbB
(86) International application number: PCT/CN2021/142587
(87) International publication number: WO 2022/143794

(57) **Abstract**

The present invention relates to the field of bio-pharmaceuticals, and provides an anti-CLDN18.2 antibody, a preparation method therefor and the use thereof. The antibody of the present invention or an antigen-binding fragment thereof can specifically bind to CLDN18.2 with a strong binding capacity. After humanization, the antibody or the antigen-binding fragment has a strong ADCC effect and CDC activity, thereby having good pharmaceutical prospects.

## Description

### TECHNICAL FIELD

The present invention relates to the field of bio-pharmaceuticals, and provides an anti-CLDN18.2 antibody and a preparation method therefor and use thereof.

### BACKGROUND

Claudins are integral membrane proteins in tight junctions of the epithelium and the endothelium. Claudin18 (CLDN18) molecules are integral transmembrane proteins (tetraspanins) with four transmembrane hydrophobic regions and two extracellular loops. CLDN18 exists in two different splice variants: CLDN18.1 and CLDN18.2. The splice variants CLDN18.1 and CLDN18.2 differ in the N-terminal portion comprising the first transmembrane (TM) region and loop 1, while the primary sequences of the protein of the C-terminal are identical.

CLDN18.1 is selectively expressed in normal lung cells, while CLDN18.2 is expressed only in normal gastric cells and the expression is restricted to differentiated gastric epithelial short-lived cells. CLDN18.2 was also found to be expressed in a variety of tumor tissues.

Due to the differential expression of CLDN18.2 between cancer cells and normal cells, CLDN18.2 becomes a target for cancer immunotherapy: the anti-CLDN18.2 antibody is capable of binding to CLDN18.2 and efficiently inducing and killing cancer cells. Although there are a variety of anti-CLDN18.2 antibodies available at present, there is still an urgent need in the art for effective anti-CLDN18.2 antibodies.

### SUMMARY

The present invention provides an anti-CLDN18.2 antibody that specifically binds to CLDN18.2.

In a first aspect, the present invention provides an anti-CLDN18.2 antibody or an antigen-binding fragment thereof, the antibody or the antigen-binding fragment thereof comprising a heavy chain variable region and a light chain variable region,
wherein the heavy chain variable region comprises 1, 2, or 3 CDRs as follows:
a VH CDR1 comprising or consisting of an amino acid sequence: SYYIH (SEQ ID NO: 41), DYGMH (SEQ ID NO: 44), NTYIY (SEQ ID NO: 47), DYYMH (SEQ ID NO: 52), TSGMS (SEQ ID NO: 55), or GYWIE (SEQ ID NO: 58);
a VH CDR2 comprising or consisting of an amino acid sequence: WIYPGSGNTKYNEKFKG (SEQ ID NO: 42), YISRGRSTTYSTDTVKG (SEQ ID NO: 45), RIDPANGNTKYAPKFQG (SEQ ID NO: 48), YISSGRSTIYSADTVKG (SEQ ID NO: 50), EIYPGSGNTYYNEKFKG (SEQ ID NO: 53), LINTYSGVPTYADDFKG (SEQ ID NO: 56), or EILPGSGSTNYNEKFKG (SEQ ID NO: 59); and
a VH CDR3 comprising or consisting of an amino acid sequence: GMDYGNYYLDY (SEQ ID NO: 43), GSYYGNAMDY (SEQ ID NO: 46), SPAYYINYYAMDY (SEQ ID NO: 49), GGYYGNAMDY (SEQ ID NO: 51), GGDYYDYDGTGYYAMDY (SEQ ID NO: 54), WSRAWFPY (SEQ ID NO: 57), or APLEGLRSGFAY (SEQ ID NO: 60); and
the light chain variable region comprises 1, 2, or 3 CDRs as follows:
   a VL CDR1 comprising or consisting of an amino acid sequence: KASQDVGTAVA (SEQ ID NO: 61), HASQNINVWLS (SEQ ID NO: 64), SASSSVSYMH (SEQ ID NO: 67), KASQNVGTNVA (SEQ ID NO: 70), KSSQSLLNSGNQRNYLT (SEQ ID NO: 73), SASSSVSSSYLY (SEQ ID NO: 76), RASESVDSYGNSFMH (SEQ ID NO: 79), or RASQSISDYLH (SEQ ID NO: 82);
   a VL CDR2 comprising or consisting of an amino acid sequence: WASTRHT (SEQ ID NO: 62), KASNLHT (SEQ ID NO: 65), DTSKLAS (SEQ ID NO: 68), STSYRYS (SEQ ID NO: 71), WASTRES (SEQ ID NO: 74), STSNLAS (SEQ ID NO: 77), RASNLES (SEQ ID NO: 80), or YASQSIS (SEQ ID NO: 83); and a VL CDR3 comprising or consisting of an amino acid sequence: QQYSSYLT (SEQ ID NO: 63), QQGQSYPYT (SEQ ID NO: 66), QQWSSNPFT (SEQ ID NO: 69), QQYNSYPLT (SEQ ID NO: 72), QSAYSYPFT (SEQ ID NO: 75), HQWSSYPPT (SEQ ID NO: 78), QQSNEDPRT (SEQ ID NO: 81), or QNGHSFPYT (SEQ ID NO: 84).

In one embodiment, the heavy chain variable region of the antibody or the antigen-binding fragment thereof comprises 3 CDRs as described above, and the light chain variable region of the antibody or the antigen-binding fragment thereof comprises 3 CDRs as described above.

In one embodiment, the heavy chain variable region of the antibody or the antigen-binding fragment thereof comprises or consists of the following CDRs:
VH CDR1: SYYIH (SEQ ID NO: 41);
VH CDR2: WIYPGSGNTKYNEKFKG (SEQ ID NO: 42);
VH CDR3: GMDYGNYYLDY (SEQ ID NO: 43); or
VH CDR1: DYGMH (SEQ ID NO: 44);
VH CDR2: YISRGRSTTYSTDTVKG (SEQ ID NO: 45);
VH CDR3: GSYYGNAMDY (SEQ ID NO: 46); or
VH CDR1: NTYIY (SEQ ID NO: 47);
VH CDR2: RIDPANGNTKYAPKFQG (SEQ ID NO: 48);
VH CDR3: SPAYYINYYAMDY (SEQ ID NO: 49); or
VH CDR1: DYGMH (SEQ ID NO: 44);
VH CDR2: YISSGRSTIYSADTVKG (SEQ ID NO: 50);
VH CDR3: GGYYGNAMDY (SEQ ID NO: 51); or
VH CDR1: DYYMH (SEQ ID NO: 52);
VH CDR2: EIYPGSGNTYYNEKFKG (SEQ ID NO: 53);
VH CDR3: GGDYYDYDGTGYYAMDY (SEQ ID NO: 54); or
VH CDR1: TSGMS (SEQ ID NO: 55);
VH CDR2: LINTYSGVPTYADDFKG (SEQ ID NO: 56);
VH CDR3: WSRAWFPY (SEQ ID NO: 57); or
VH CDR1: GYWIE (SEQ ID NO: 58);
VH CDR2: EILPGSGSTNYNEKFKG (SEQ ID NO: 59);
VH CDR3: APLEGLRSGFAY (SEQ ID NO: 60).

In one embodiment, the light chain variable region of the antibody or the antigen-binding fragment thereof comprises or consists of the following CDRs:
VL CDR1: KASQDVGTAVA (SEQ ID NO: 61);
VL CDR2: WASTRHT (SEQ ID NO: 62);
VL CDR3: QQYSSYLT (SEQ ID NO: 63); or
VL CDR1: HASQNINVWLS (SEQ ID NO: 64);
VL CDR2: KASNLHT (SEQ ID NO: 65);
VL CDR3: QQGQSYPYT (SEQ ID NO: 66); or
VL CDR1: SASSSVSYMH (SEQ ID NO: 67);
VL CDR2: DTSKLAS (SEQ ID NO: 68);
VL CDR3: QQWSSNPFT (SEQ ID NO: 69); or
VL CDR1: KASQNVGTNVA (SEQ ID NO: 70);
VL CDR2: STSYRYS (SEQ ID NO: 71);
VL CDR3: QQYNSYPLT (SEQ ID NO: 72); or
VL CDR1: KSSQSLLNSGNQRNYLT (SEQ ID NO: 73);
VL CDR2: WASTRES (SEQ ID NO: 74);
VL CDR3: QSAYSYPFT (SEQ ID NO: 75); or
VL CDR1: SASSSVSSSYLY (SEQ ID NO: 76);
VL CDR2: STSNLAS (SEQ ID NO: 77);
VL CDR3: HQWSSYPPT (SEQ ID NO: 78); or
VL CDR1: RASESVDSYGNSFMH (SEQ ID NO: 79);
VL CDR2: RASNLES (SEQ ID NO: 80);
VL CDR3: QQSNEDPRT (SEQ ID NO: 81); or
VL CDR1: RASQSISDYLH (SEQ ID NO: 82);
VL CDR2: YASQSIS (SEQ ID NO: 83);
VL CDR3: QNGHSFPYT (SEQ ID NO: 84).

In one embodiment, the antibody or the antigen-binding fragment thereof comprises a heavy chain variable region and a light chain variable region, wherein the heavy chain variable region comprises or consists of a VH CDR1 set forth in SEQ ID NO: 41, a VH CDR2 set forth in SEQ ID NO: 42, and a VH CDR3 set forth in SEQ ID NO: 43, and the light chain variable region comprises or consists of a VL CDR1 set forth in SEQ ID NO: 61, a VL CDR2 set forth in SEQ ID NO: 62, and a VL CDR3 set forth in SEQ ID NO: 63.

In one embodiment, the antibody or the antigen-binding fragment thereof comprises a heavy chain variable region and a light chain variable region, wherein the heavy chain variable region comprises or consists of a VH CDR1 set forth in SEQ ID NO: 41, a VH CDR2 set forth in SEQ ID NO: 42, and a VH CDR3 set forth in SEQ ID NO: 43, and the light chain variable region comprises or consists of a VL CDR1 set forth in SEQ ID NO: 64, a VL CDR2 set forth in SEQ ID NO: 65, and a VL CDR3 set forth in SEQ ID NO: 66.

In one embodiment, the antibody or the antigen-binding fragment thereof comprises a heavy chain variable region and a light chain variable region, wherein the heavy chain variable region comprises or consists of a VH CDR1 set forth in SEQ ID NO: 44, a VH CDR2 set forth in SEQ ID NO: 45, and a VH CDR3 set forth in SEQ ID NO: 46, and the light chain variable region comprises or consists of a VL CDR1 set forth in SEQ ID NO: 67, a VL CDR2 set forth in SEQ ID NO: 68, and a VL CDR3 set forth in SEQ ID NO: 69.

In one embodiment, the antibody or the antigen-binding fragment thereof comprises a heavy chain variable region and a light chain variable region, wherein the heavy chain variable region comprises or consists of a VH CDR1 set forth in SEQ ID NO: 44, a VH CDR2 set forth in SEQ ID NO: 45, and a VH CDR3 set forth in SEQ ID NO: 46, and the light chain variable region comprises or consists of a VL CDR1 set forth in SEQ ID NO: 70, a VL CDR2 set forth in SEQ ID NO: 71, and a VL CDR3 set forth in SEQ ID NO: 72.

In one embodiment, the antibody or the antigen-binding fragment thereof comprises a heavy chain variable region and a light chain variable region, wherein the heavy chain variable region comprises or consists of a VH CDR1 set forth in SEQ ID NO: 44, a VH CDR2 set forth in SEQ ID NO: 45, and a VH CDR3 set forth in SEQ ID NO: 46, and the light chain variable region comprises or consists of a VL CDR1 set forth in SEQ ID NO: 73, a VL CDR2 set forth in SEQ ID NO: 74, and a VL CDR3 set forth in SEQ ID NO: 75.

In one embodiment, the antibody or the antigen-binding fragment thereof comprises a heavy chain variable region and a light chain variable region, wherein the heavy chain variable region comprises or consists of a VH CDR1 set forth in SEQ ID NO: 47, a VH CDR2 set forth in SEQ ID NO: 48, and a VH CDR3 set forth in SEQ ID NO: 49, and the light chain variable region comprises or consists of a VL CDR1 set forth in SEQ ID NO: 67, a VL CDR2 set forth in SEQ ID NO: 68, and a VL CDR3 set forth in SEQ ID NO: 69.

In one embodiment, the antibody or the antigen-binding fragment thereof comprises a heavy chain variable region and a light chain variable region, wherein the heavy chain variable region comprises or consists of a VH CDR1 set forth in SEQ ID NO: 47, a VH CDR2 set forth in SEQ ID NO: 48, and a VH CDR3 set forth in SEQ ID NO: 49, and the light chain variable region comprises or consists of a VL CDR1 set forth in SEQ ID NO: 70, a VL CDR2 set forth in SEQ ID NO: 71, and a VL CDR3 set forth in SEQ ID NO: 72.

In one embodiment, the antibody or the antigen-binding fragment thereof comprises a heavy chain variable region and a light chain variable region, wherein the heavy chain variable region comprises or consists of a VH CDR1 set forth in SEQ ID NO: 47, a VH CDR2 set forth in SEQ ID NO: 48, and a VH CDR3 set forth in SEQ ID NO: 49, and the light chain variable region comprises or consists of a VL CDR1 set forth in SEQ ID NO: 73, a VL CDR2 set forth in SEQ ID NO: 74, and a VL CDR3 set forth in SEQ ID NO: 75.

In one embodiment, the antibody or the antigen-binding fragment thereof comprises a heavy chain variable region and a light chain variable region, wherein the heavy chain variable region comprises or consists of a VH CDR1 set forth in SEQ ID NO: 44, a VH CDR2 set forth in SEQ ID NO: 50, and a VH CDR3 set forth in SEQ ID NO: 51, and the light chain variable region comprises or consists of a VL CDR1 set forth in SEQ ID NO: 76, a VL CDR2 set forth in SEQ ID NO: 77, and a VL CDR3 set forth in SEQ ID NO: 78.

In one embodiment, the antibody or the antigen-binding fragment thereof comprises a heavy chain variable region and a light chain variable region, wherein the heavy chain variable region comprises or consists of a VH CDR1 set forth in SEQ ID NO: 52, a VH CDR2 set forth in SEQ ID NO: 53, and a VH CDR3 set forth in SEQ ID NO: 54, and the light chain variable region comprises or consists of a VL CDR1 set forth in SEQ ID NO: 76, a VL CDR2 set forth in SEQ ID NO: 77, and a VL CDR3 set forth in SEQ ID NO: 78.

In one embodiment, the antibody or the antigen-binding fragment thereof comprises a heavy chain variable region and a light chain variable region, wherein the heavy chain variable region comprises or consists of a VH CDR1 set forth in SEQ ID NO: 55, a VH CDR2 set forth in SEQ ID NO: 56, and a VH CDR3 set forth in SEQ ID NO: 57, and the light chain variable region comprises or consists of a VL CDR1 set forth in SEQ ID NO: 79, a VL CDR2 set forth in SEQ ID NO: 80, and a VL CDR3 set forth in SEQ ID NO: 81.

In one embodiment, the antibody or the antigen-binding fragment thereof comprises a heavy chain variable region and a light chain variable region, wherein the heavy chain variable region comprises or consists of a VH CDR1 set forth in SEQ ID NO: 55, a VH CDR2 set forth in SEQ ID NO: 56, and a VH CDR3 set forth in SEQ ID NO: 57, and the light chain variable region comprises or consists of a VL CDR1 set forth in SEQ ID NO: 82, a VL CDR2 set forth in SEQ ID NO: 83, and a VL CDR3 set forth in SEQ ID NO: 84.

In one embodiment, the antibody or the antigen-binding fragment thereof comprises a heavy chain variable region and a light chain variable region, wherein the heavy chain variable region comprises or consists of a VH CDR1 set forth in SEQ ID NO: 58, a VH CDR2 set forth in SEQ ID NO: 59, and a VH CDR3 set forth in SEQ ID NO: 60, and the light chain variable region comprises or consists of a VL CDR1 set forth in SEQ ID NO: 79, a VL CDR2 set forth in SEQ ID NO: 80, and a VL CDR3 set forth in SEQ ID NO: 81.

In one embodiment, the antibody or the antigen-binding fragment thereof comprises a heavy chain variable region and a light chain variable region, wherein the heavy chain variable region comprises or consists of a VH CDR1 set forth in SEQ ID NO: 58, a VH CDR2 set forth in SEQ ID NO: 59, and a VH CDR3 set forth in SEQ ID NO: 60, and the light chain variable region comprises or consists of a VL CDR1 set forth in SEQ ID NO: 82, a VL CDR2 set forth in SEQ ID NO: 83, and a VL CDR3 set forth in SEQ ID NO: 84.

In one embodiment, the antibody or the antigen-binding fragment thereof is a murine antibody, a chimeric antibody, or a humanized antibody.

In one embodiment, the heavy chain variable region of the antibody or the antigen-binding fragment thereof comprises a sequence set forth in any one of SEQ ID NO: 1, SEQ ID NO: 7, SEQ ID NO: 9, SEQ ID NO: 17, SEQ ID NO: 19, SEQ ID NO: 23, and SEQ ID NO: 25, or a sequence having at least 90% identity to the sequences described above, or an amino acid sequence having one or more conservative amino acid substitutions as compared to the sequences described above.

In one embodiment, the light chain variable region of the antibody or the antigen-binding fragment thereof comprises a sequence set forth in any one of SEQ ID NO: 3, SEQ ID NO: 5, SEQ ID NO: 11, SEQ ID NO: 13, SEQ ID NO: 15, SEQ ID NO: 21, SEQ ID NO: 27, and SEQ ID NO: 29, or a sequence having at least 90% identity to the sequences described above, or an amino acid sequence having one or more conservative amino acid substitutions as compared to the sequences described above.

In one embodiment, the heavy chain variable region of the antibody or the antigen-binding fragment thereof has an amino acid sequence set forth in SEQ ID NO: 1, and the light chain variable region has an amino acid sequence set forth in SEQ ID NO: 3 or SEQ ID NO: 5.

In one embodiment, the heavy chain variable region of the antibody or the antigen-binding fragment thereof has an amino acid sequence set forth in SEQ ID NO: 7 or SEQ ID NO: 9, and the light chain variable region has an amino acid sequence set forth in SEQ ID NO: 11, SEQ ID NO: 13, or SEQ ID NO: 15.

In one embodiment, the heavy chain variable region of the antibody or the antigen-binding fragment thereof has an amino acid sequence set forth in SEQ ID NO: 17 or SEQ ID NO: 19, and the light chain variable region has an amino acid sequence set forth in SEQ ID NO: 21.

In one embodiment, the heavy chain variable region of the antibody or the antigen-binding fragment thereof has an amino acid sequence set forth in SEQ ID NO: 23 or SEQ ID NO: 25, and the light chain variable region has an amino acid sequence set forth in SEQ ID NO: 27 or SEQ ID NO: 29.

In one embodiment, the heavy chain variable region of the antibody or the antigen-binding fragment thereof has an amino acid sequence set forth in SEQ ID NO: 1, and the light chain variable region has an amino acid sequence set forth in SEQ ID NO: 3. In one embodiment, the heavy chain variable region of the antibody or the antigen-binding fragment thereof has an amino acid sequence set forth in SEQ ID NO: 7, and the light chain variable region has an amino acid sequence set forth in SEQ ID NO: 15. In one embodiment, the heavy chain variable region of the antibody or the antigen-binding fragment thereof has an amino acid sequence set forth in SEQ ID NO: 9, and the light chain variable region has an amino acid sequence set forth in SEQ ID NO: 11. In one embodiment, the heavy chain variable region of the antibody or the antigen-binding fragment thereof has an amino acid sequence set forth in SEQ ID NO: 9, and the light chain variable region has an amino acid sequence set forth in SEQ ID NO: 13. In one embodiment, the heavy chain variable region of the antibody or the antigen-binding fragment thereof has an amino acid sequence set forth in SEQ ID NO: 9, and the light chain variable region has an amino acid sequence set forth in SEQ ID NO: 15. In one embodiment, the heavy chain variable region of the antibody or the antigen-binding fragment thereof has an amino acid sequence set forth in SEQ ID NO: 17, and the light chain variable region has an amino acid sequence set forth in SEQ ID NO: 21. In one embodiment, the heavy chain variable region of the antibody or the antigen-binding fragment thereof has an amino acid sequence set forth in SEQ ID NO: 19, and the light chain variable region has an amino acid sequence set forth in SEQ ID NO: 21. In one embodiment, the heavy chain variable region of the antibody or the antigen-binding fragment thereof has an amino acid sequence set forth in SEQ ID NO: 25, and the light chain variable region has an amino acid sequence set forth in SEQ ID NO: 27.

In one embodiment, the antibody is a humanized antibody. In one embodiment, the framework region of the variable region of the heavy chain and the light chain of the antibody is humanized.

In one embodiment, the heavy chain variable region of the antibody or the antigen-binding fragment thereof comprises a sequence set forth in SEQ ID NO: 31 or SEQ ID NO: 33, or a sequence having at least 90% identity to the sequences described above, or an amino acid sequence having one or more conservative amino acid substitutions as compared to the sequences described above; and/or
the light chain variable region of the antibody or the antigen-binding fragment thereof comprises a sequence set forth in SEQ ID NO: 35, or a sequence having at least 90% identity to the sequence described above, or an amino acid sequence having one or more conservative amino acid substitutions as compared to the sequence described above.

In one embodiment, the heavy chain variable region of the antibody or the antigen-binding fragment thereof has an amino acid sequence set forth in SEQ ID NO: 31, and the light chain variable region has an amino acid sequence set forth in SEQ ID NO: 35. In one embodiment, the heavy chain variable region of the antibody or the antigen-binding fragment has an amino acid sequence set forth in SEQ ID NO: 33, and the light chain variable region has an amino acid sequence set forth in SEQ ID NO: 35.

In some embodiments, the antibody or the antigen-binding fragment thereof further comprises a heavy chain constant region, a light chain constant region, an Fc region, or a combination thereof. In some embodiments, the light chain is of kappa (κ) or lambda (λ) type. In some embodiments, the light chain constant region is a κ or λ chain constant region. In some embodiments, the antibody or the antigen-binding fragment is of one of the isotypes IgG, IgM, IgA, IgE, and IgD. In some embodiments, the isotype is IgG1, IgG2, IgG3, or IgG4. In some embodiments, the antibody is an IgG1 antibody.

In some embodiments, the Fc is a variant Fc region. In some embodiments, the variant Fc region has one or more amino acid modifications, such as substitutions, deletions, or insertions, relative to a parent Fc region. In some embodiments, the amino acid modification of the Fc region alters effector function activity, relative to the activity of the parent Fc region. In some embodiments, the variant Fc region can have altered (i.e., increased or decreased) antibody-dependent cellular cytotoxicity (ADCC), complement-dependent cytotoxicity (CDC), phagocytosis, opsonization, or cell binding. In some embodiments, the amino acid modification of the Fc region can alter the affinity of the variant Fc region for an FcyR (Fcy receptor), relative to the parent Fc region. In some embodiments, the Fc region is derived from IgG1 or IgG4. In some embodiments, a mutation of the Fc region is N297A.

In some embodiments, the antibody or the antigen-binding fragment thereof is an isolated antibody or an antigen-binding fragment. In some embodiments, the antibody or the antigen-binding fragment thereof is an scFV, an Fab, an F(ab)₂, or an IgG1. In some embodiments, the antibody or the antigen-binding fragment thereof is a monoclonal antibody.

In some embodiments, the antibody or the antigen-binding fragment thereof is a chimeric antibody. In one embodiment, the antibody is CH239H-2-K-6, comprising a heavy chain variable region having an amino acid sequence set forth in SEQ ID NO: 7, a heavy chain constant region having an amino acid sequence set forth in SEQ ID NO: 37, a light chain variable region having an amino acid sequence set forth in SEQ ID NO: 15, and a light chain constant region having an amino acid sequence set forth in SEQ ID NO: 39.

In one embodiment, the antibody is CH239H-9-K-4, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 13, and a light chain constant region having an amino acid sequence set forth in SEQ ID NO: 39.

In one embodiment, the antibody is CH394H2-K-1, comprising a heavy chain variable region having an amino acid sequence set forth in SEQ ID NO: 25, a heavy chain constant region having an amino acid sequence set forth in SEQ ID NO: 37, a light chain variable region having an amino acid sequence set forth in SEQ ID NO: 27, and a light chain constant region having an amino acid sequence set forth in SEQ ID NO: 39.

In one embodiment, the antibody is CH372H6-K-1, comprising a heavy chain variable region having an amino acid sequence set forth in SEQ ID NO: 19, a heavy chain constant region having an amino acid sequence set forth in SEQ ID NO: 37, a light chain variable region having an amino acid sequence set forth in SEQ ID NO: 21, and a light chain constant region having an amino acid sequence set forth in SEQ ID NO: 39.

In one embodiment, the antibody is CH239H-9-K-6, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 15, and a light chain constant region having an amino acid sequence set forth in SEQ ID NO: 39.

In one embodiment, the antibody is CH239H-9-K-1, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 11, and a light chain constant region having an amino acid sequence set forth in SEQ ID NO: 39.

In one embodiment, the antibody is CH101H1-K-1, comprising a heavy chain variable region having an amino acid sequence set forth in SEQ ID NO: 1, a heavy chain constant region having an amino acid sequence set forth in SEQ ID NO: 37, a light chain variable region having an amino acid sequence set forth in SEQ ID NO: 3, and a light chain constant region having an amino acid sequence set forth in SEQ ID NO: 39.

In one embodiment, the antibody is CH372H1-K-1, comprising a heavy chain variable region having an amino acid sequence set forth in SEQ ID NO: 17, a heavy chain constant region having an amino acid sequence set forth in SEQ ID NO: 37, a light chain variable region having an amino acid sequence set forth in SEQ ID NO: 21, and a light chain constant region having an amino acid sequence set forth in SEQ ID NO: 39.

In some embodiments, the antibody is a humanized antibody. In one embodiment, the antibody is H239H-2a-K-6a, comprising a heavy chain variable region having an amino acid sequence set forth in SEQ ID NO: 31, a heavy chain constant region having an amino acid sequence set forth in SEQ ID NO: 37, a light chain variable region having an amino acid sequence set forth in SEQ ID NO: 35, and a light chain constant region having an amino acid sequence set forth in SEQ ID NO: 39. In one embodiment, the antibody comprises a sequence having at least 80% identity to the antibody H239H-2a-K-6a, or an amino acid sequence having one or more conservative amino acid substitutions as compared to the sequence described above.

In one embodiment, the antibody is H239H-2b-K-6a, comprising a heavy chain variable region having an amino acid sequence set forth in SEQ ID NO: 33, a heavy chain constant region having an amino acid sequence set forth in SEQ ID NO: 37, a light chain variable region having an amino acid sequence set forth in SEQ ID NO: 35, and a light chain constant region having an amino acid sequence set forth in SEQ ID NO: 39. In one embodiment, the antibody comprises a sequence having at least 80% identity to the Antibody H239H-2b-K-6a, or an amino acid sequence having one or more conservative amino acid substitutions as compared to the sequence described above.

In some embodiments, the antibody described herein comprises two heavy chains with the same sequence and two light chains with the same sequence.

In a second aspect, the present invention provides a nucleic acid molecule comprising a nucleotide encoding the antibody or the antigen-binding fragment thereof of the present invention. In some embodiments, the nucleic acid molecule is an isolated nucleic acid molecule.

In some embodiments, the nucleic acid molecule encodes the heavy chain variable region of the antibody, and the nucleic acid molecule has a nucleotide sequence set forth in SEQ ID NO: 2, SEQ ID NO: 8, SEQ ID NO: 10, SEQ ID NO: 18, SEQ ID NO: 20, SEQ ID NO: 24, SEQ ID NO: 26, SEQ ID NO: 32, or SEQ ID NO: 34, or a sequence having at least 90% identity to the sequences described above.

In some embodiments, the nucleic acid molecule encodes the light chain variable region of the antibody, and the nucleic acid molecule has a nucleotide sequence set forth in SEQ ID NO: 4, SEQ ID NO: 6, SEQ ID NO: 12, SEQ ID NO: 14, SEQ ID NO: 16, SEQ ID NO: 22, SEQ ID NO: 28, SEQ ID NO: 30, or SEQ ID NO: 36, or a sequence having at least 90% identity to the sequences described above.

In some embodiments, the nucleic acid molecule encodes the heavy chain variable region and/or the light chain variable region of the antibody, and the nucleotide sequences of the heavy chain variable region and the light chain variable region are as described above.

In a third aspect, the present invention provides a biomaterial, which is:
(1) a vector, a host cell, or a microorganism, or the like, comprising the nucleic acid molecule of the present invention; or
(2) an expression product, a suspension, a supernatant, or the like, of the (1) described above.

In some embodiments, the vector, the host cell, or the microorganism is an isolated vector, host cell, or microorganism. In some embodiments, the host cell is a CHO cell, an HEK cell (e.g., an HEK293F cell), a BHK cell, a Cos1 cell, a Cos7 cell, a CV1 cell, or a murine L cell. In some embodiments, the host cell is a CHO cell.

In a fourth aspect, provided is a composition comprising the antibody or the antigen-binding fragment thereof of the present invention. In some embodiments, the composition is a pharmaceutical composition further comprising a pharmaceutically acceptable carrier.

In a fifth aspect, provided is a method for preparing the antibody or the antigen-binding fragment thereof of the present invention, comprising: culturing the host cell described above to express the antibody or the antigen-binding fragment and isolating the antibody or the antigen-binding fragment thereof.

In a sixth aspect, provided are a therapeutic method and use. In some embodiments, provided is a method for treating or ameliorating a cancer or a tumor, wherein the method comprises administering to a patient an effective dose of the antibody or the antigen-binding fragment thereof. In some embodiments, provided is use of the antibody or the antigen-binding fragment thereof in treating or ameliorating a cancer or a tumor. In some embodiments, provided is use of the antibody or the antigen-binding fragment thereof in the manufacture of a medicament for treating or ameliorating a cancer or a tumor.

In some embodiments, the cancer or the tumor includes, but is not limited to, bladder cancer, ovarian cancer (e.g., ovarian adenocarcinoma and ovarian teratocarcinoma), lung cancer (e.g., small cell lung cancer and non-small cell lung cancer), adenocarcinoma, gastric cancer, breast cancer, liver cancer, pancreatic cancer, skin cancer (e.g., basal cell carcinoma and squamous cell carcinoma), malignant melanoma, head and neck cancer, sarcoma (e.g., synovial sarcoma and carcinosarcoma), bile duct cancer, renal cancer (clear cell renal cell carcinoma and papillary renal cell carcinoma), colon cancer, small intestine cancer, testicular embryonal carcinoma, placental choriocarcinoma, cervical cancer, testicular cancer, uterine cancer, esophageal cancer, gallbladder cancer cells, or the like.

In a seventh aspect, provided are a diagnostic method and use. In some embodiments, provided is a method for detecting CLDN18.2 expression in a sample, wherein the method comprises contacting the sample with the antibody or the antigen-binding fragment thereof such that the antibody or the antigen-binding fragment thereof binds to CLDN18.2, and detecting the binding, i.e., an amount of CLD18.2 in the sample. In some embodiments, provided is use of the antibody or the antigen-binding fragment thereof in the manufacture of a kit for the diagnosis or prognosis of a cancer or a tumor. In some embodiments, provided is a diagnostic or prognostic kit comprising the antibody or the antigen-binding fragment thereof.

The antibody or the antigen-binding fragment thereof of the present invention can specifically bind to CLDN18.2 and has strong binding ability. After being humanized, the antibody has strong ADCC effect and CDC activity, and has good druggability.

### BRIEF DESCRIPTION OF THE DRAWINGS

FIG. 1 shows the flow cytometry assay results for CHO-CLDN18.2 cells, CHO cells and CHO-CLDN18.2 cells being depicted from left to right in the figure;
FIG. 2 shows the results of DotBlot assay for CHO-CLDN18.1 cells;
FIG. 3 shows the flow cytometry assay results for clones of 4 strains of cells that specifically bind to CLDN18.2 and do not bind to CLDN18.1;
FIG. 4 shows a dose curve of anti-CLDN18.2 chimeric antibody binding to KATOIII cells with the ordinate indicating the mean fluorescence intensity;
FIG. 5 is the result of the cell binding specificity of the anti-CLDN18.2 humanized antibody, wherein H239H-2a+K-6a represents the Antibody H239H-2a-K-6a, and H239H-2b+K-6a represents the Antibody H239H-2b-K-6a.

### DETAILED DESCRIPTION

The present invention will be illustrated with reference to specific embodiments. The reagents and instruments used in the following methods are those conventional in the art and commercially available unless otherwise specified; the methods used are all conventional in the art, and those skilled in the art can undoubtedly implement the methods and obtain the corresponding results according to the content described in the embodiments.

### Antibodies and nucleotides

In the present invention, "antibody" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. Embodiments of the antibody include, but are not limited to, complementarity determining regions (CDRs) of a heavy chain, a light chain, or a ligand binding portion thereof, heavy chain variable regions (VHs), light chain variable regions (VLs), heavy chain constant regions (CHs), light chain constant regions (CLs), framework regions (FRs), or any portion thereof, or at least a portion of a binding protein. The CDRs include light chain CDRs (VL CDR1-3) and heavy chain CDRs (VH CDR1-3).

As used herein, the term "antibody fragment" or "antigen-binding fragment" is a part of an antibody, such as F(ab')₂, F(ab)₂, Fab', Fab, Fv, scFv, etc. Regardless of its structure, the antigen-binding fragment binds to the same antigen recognized by an intact antibody. The term "antigen-binding fragment" includes aptamers, mirror image isomers, and bivalent antibodies. The term "antigen-binding fragment" also includes any synthetic or genetically engineered protein that functions as an antibody by binding to a specific antigen to form a complex.

The term "antibody" includes a wide variety of polypeptides that can be biochemically distinguished. It will be understood by those skilled in the art that the classes of heavy chains include gamma, mu, alpha, delta, or epsilon (γ, µ, α, δ, or ε), with some subclasses (e.g., γ1-γ4). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. Each modified form of these classes and isotypes will be readily conceivable for those of ordinary skills in the art and therefore fall within the scope of the present disclosure, and all immunoglobulin classes are within the scope of the present disclosure. With respect to IgG, a standard immunoglobulin molecule comprises two identical light chain polypeptides having a molecular weight of about 23,000 Daltons and two identical heavy chain polypeptides having a molecular weight of about 53,000-70,000 Daltons. Typically, these four chains are connected in a "Y" configuration through disulfide bonds, wherein the light chains start at the opening of "Y" configuration and extend through the variable region to surround the heavy chains.

Light chains can be classified into kappa or lambda (κ or λ). Each heavy chain may be connected with a κ or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; the immunoglobulin λ light chain variable region is V_{λ}.

The light and heavy chains can be divided into structurally and functionally homologous regions. The terms "constant" and "variable" are used in accordance with function. The light chain variable region (VL) and the heavy chain variable region (VH) determine the antigen recognition and specificity; the light chain constant region (CL) and the heavy chain constant regions (e.g., CH1, CH2, or CH3) impart important biological properties such as secretion, transplacental movement, Fc receptor binding, and complement fixation. By convention, the numbering of amino acids in the constant regions increases as they become further away from the antigen-combining site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous amino acid sequences that are specifically positioned to form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of the β-sheet structure. Thus, the framework regions position the CDRs in a correct orientation by interchain non-covalent interactions through forming a scaffold. The antigen-binding domain formed by the specifically positioned CDRs defines a surface complementary to an epitope on the immunoreactive antigen that facilitates the non-covalent binding of the antibody to its cognate epitope. For any given heavy or light chain variable region, amino acids comprising the CDRs and the framework regions may be identified by one of ordinary skills in the art according to known methods, as they have been precisely defined (see Sequences of Proteins of Immunological Interest, Kabat, E., et al., U.S. Department of Health and Human Services, (1983) and Chothia and Lesk, J. Mol. Biol., 196:901-917 (1987)).

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-combining sites found in the variable regions of heavy and light chain polypeptides. This specific region is described in Kabat et al., U.S. Dept. of Health and Human Services, "Sequences of Proteins of Immunological Interest" (1983) and Chothia et al., J. Mol. Biol. 196:901-917 (1987), which are incorporated herein by reference in their entireties. CDRs defined according to Kabat and Chothia include overlaps or subsets of amino acid residues when compared to each other. Nevertheless, it is within the scope of the present invention to apply any definition to refer to the CDRs of an antibody or a variant thereof. The exact number of residues comprising a particular CDR will vary according to the sequence and size of the CDR. Those skilled in the art can generally determine which particular residues a CDR comprises based on the variable region amino acid sequence of an antibody.

Kabat et al. also defined a numbering system applicable to the variable region sequence of any antibody. Those of ordinary skills in the art can apply the "Kabat numbering" system to any variable region sequence without depending on other experimental data beyond the sequence itself. "Kabat numbering" refers to the numbering system proposed by Kabat et al., U.S. Dept. of Health and Human Services in "Sequence of Proteins of Immunological Interest" (1983). EU or Chothia numbering system can be also applicable to the antibody.

The antibodies of the present invention may be derived from any animal, including birds and mammals. Preferably, the antibody is derived from a human, a mouse, a donkey, a rabbit, a goat, a camel, a llama, a horse, or a chicken origin. In another embodiment, the variable region may be derived from a condricthoid origin (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant region of the antibody may be derived from different immunoglobulin molecules. For example, the heavy chain constant region of a polypeptide may comprise a CH1 domain derived from an IgG₁ molecule and a hinge region derived from an IgG₃ molecule. In another embodiment, the heavy chain constant region may comprise a hinge region derived partially from an IgG₁ molecule and partially from an IgG₃ molecule. In another embodiment, a portion of the heavy chain may comprise a chimeric hinge region derived partially from an IgG₁ molecule and partially from an IgG₄ molecule. In the present invention, the term "light chain constant region" includes a part of amino acid sequence from the light chain of an antibody. Preferably, the light chain constant region comprises at least one of a constant κ domain or a constant λ domain.

"Light chain-heavy chain pair" refers to a collection of light and heavy chains that can form dimers through disulfide bonds between the CL domain of the light chain and the CH1 domain of the heavy chain.

"VH domain" includes the variable domain at the amino terminus of an immunoglobulin heavy chain, and the term "CH1 domain" includes the first constant region (mostly at the amino terminus) of an immunoglobulin heavy chain. In an intact native IgG molecule, each N297 in the two CH2 domains is attached to one branched carbohydrate chain. The CH3 domain extends from the CH2 domain to the C terminus of the IgG molecule and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified in the present invention) is derived from a second species. In certain embodiments, the variable region is derived from a non-human origin (e.g., mouse or primate) and the constant region is derived from a human origin.

"Specifically bind to" or "specific for" generally refers to that an antibody or an antigen-binding fragment and a specific antigen bind to an epitope through its antigen-binding domain to form a relatively stable complex. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to Antigen a include antibodies that have an equilibrium dissociation constant KD of less than or equal to about 100 nM, or less than or equal to about 50 nM with Antigen a.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody, the antigen-binding fragment, or the pharmaceutical composition of the present invention for detection, diagnostic procedures, and/or treatment.

It should be noted that the term "an" entity refers to one or more of the entities. For example, "an antibody" should be interpreted as one or more antibodies. Thus, the terms "a" (or "an"), "one or more", and "at least one" are be used interchangeably herein.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence, and it may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The fact that an amino acid is encoded by different codons is known as "codon degeneracy".

Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the substitution of one amino acid residue with another amino acid residue with a side chain (R group) of similar chemical properties (e.g., charge or hydrophobicity). Generally, conservative amino acid substitutions do not substantially alter the functional properties of the protein. Examples of amino acid classes with a side chain of similar chemical properties include: 1) amino acids with an aliphatic side chain: glycine, alanine, valine, leucine, and isoleucine; 2) amino acids with an aliphatic hydroxyl side chain: serine and threonine; 3) amino acids with an amide-containing side chain: asparagine and glutamine; 4) amino acids with an aromatic side chain: phenylalanine, tyrosine, and tryptophan; 5) amino acids with a basic side chain: lysine, arginine, and histidine; and 6) amino acids with an acidic side chain: aspartic acid and glutamic acid.

The number of amino acids of "conservative amino acid substitutions of VL or VH" is about 1, about 2, about 3, about 4, about 5, about 6, about 8, about 9, about 10, about 11, about 13, about 14, about 15 conservative amino acid substitutions, or a range between any two of these values (inclusive) or any value therein. The number of amino acids of "conservative amino acid substitutions of a heavy chain constant region, a light chain constant region, a heavy chain or a light chain" is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "isolated" as used herein with respect to cells, nucleic acids, polypeptides, antibodies and the like, e.g., "isolated" DNA, RNA, polypeptides, or antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell culture media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides and antibodies and the like is at least about 50%, about 60%, about 70%, about 80%, about 90%, about 95%, about 99%, or a range between any two of these values (inclusive) or any value therein.

The term "recombinant", with regard to a polypeptide or polynucleotide, refers to a polypeptide or polynucleotide that does not occur in nature, and non-limiting examples can be combined to produce a polynucleotide or polypeptide that does not normally occur.

"Homology" or "identity" or "similarity" refers to sequence similarity between two peptides or between two nucleic acid molecules. Homology can be determined by comparing the positions that can be aligned in the sequences. When a position of the compared sequences is occupied by the same base or amino acid, the molecules are homologous at that position. The degree of homology between the sequences is a function of the number of matching or homologous positions shared by the sequences.

"At least 80% identity" is about 80% identity, about 81% identity, about 82% identity, about 83% identity, about 84% identity, about 85% identity, about 86% identity, about 87% identity, about 88% identity, about 89% identity, about 90% identity, about 91% identity, about 92% identity, about 93% identity, about 94% identity, about 95% identity, about 96% identity, about 97% identity, about 98% identity, about 99% identity, or a range between any two of these values (inclusive) or any value therein.

"At least 90% identity" refers to about 90% identity, about 91% identity, about 92% identity, about 93% identity, about 94% identity, about 95% identity, about 96% identity, about 97% identity, about 98% identity, about 99% identity, or a range between any two of these values (inclusive) or any value therein.

A polynucleotide or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above specified percentage identity and encoding polypeptides having identical or similar biological activity.

A polynucleotide consists of a specific sequence of four nucleotide bases: adenine (A), cytosine (C), guanine (G), thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transport RNA, ribosomal RNA, ribozymes, cDNA, dsRNA, siRNA, miRNA, recombinant polynucleotides, branched polynucleotides, plasmids, vectors, isolated DNA of any sequence, isolated RNA of any sequence, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide of the present invention include a double-stranded form and each of the two complementary single-stranded forms known or predicted to constitute the double-stranded form.

The term "encoding" as applied to a polynucleotide means that a polynucleotide known to "encode" a polypeptide, when in its native state or manipulated by methods well known to those skilled in the art, can produce the polypeptide and/or a fragment thereof by transcript and/or translation.

### Anti-CLDN18.2 antibody

In some embodiments, the antibody or the antigen-binding fragment thereof of the present invention can specifically bind to CLDN18.2. In some embodiments, the antibody or the antigen-binding fragment thereof is a murine antibody, a chimeric antibody, or a humanized antibody. In some embodiments, the antibody or the antigen-binding fragment thereof of the present invention has one or more of the following properties: a) specifically binding to CLDN18.2; b) high affinity; c) high ADCC activity; and d) high CDC activity.

In some embodiments, the antibody or the antigen-binding fragment comprises an amino acid sequence having one or more modification groups. For example, the antibody or the antigen-binding fragment of the present invention may comprise a flexible linker sequence, or may be modified to add a functional group (e.g., PEG, a drug, a toxin, or a tag).

The antibody or the antigen-binding fragment of the present invention includes modified derivatives, i.e., modified by covalent linking of any type of molecules to the antibody or the antigen-binding fragment, wherein the covalent linking does not prevent the antibody or the antigen-binding fragment from binding to an epitope. The following examples are included but not by way of limitation, wherein an antibody or an antigen-binding fragment may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In some embodiments, the antibody or the antigen-binding fragment may be conjugated with a therapeutic agent, a prodrug, a peptide, a protein, an enzyme, a virus, a lipid, a biological response modifier, an agent, or PEG.

The antibody or the antigen-binding fragment may be detectably labeled by coupling to a chemiluminescent compound. The presence of the chemiluminescent-labeled antibody or antigen-binding fragment is then determined by detecting the luminescence that occurs during the chemical reaction. Examples of chemiluminescent-labeled compounds include luminol, isoluminol, aromatic acridinium esters, imidazole, acridinium salts, and oxalate esters.

### Preparation of Antibodies

In certain embodiments, the prepared antibody does not elicit a deleterious immune response in the animal (e.g., human) to be treated. In some embodiments, the antibody, the antigen-binding fragment or the derivative thereof of the present invention is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human complementarity determining regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted with corresponding residues from the CDR donor antibody, such as residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Pat. No. 5,585,089 and Riechmann et al., Nature 332:323 (1988); which are incorporated herein by reference in their entireties). The antibody can be humanized by a variety of techniques well known in the art, such as CDR grafting (Pat. Nos. EP 239,400 and WO 91/09967, and U.S. Pat. Nos. 5,225,539, 5,530,101 and 5,585,089), repair or surface rearrangement (Pat. Nos. EP592,106 and EP519,596; Padlan, et al., Molecular Immunology 28(4/5):489-498 (1991); Studnicka et al., Protein Engineering 7(6):805-814 (1994); Roguska, et al., Proc. Natl. Acad. Sci. USA 91:969-973 (1994)), and chain rearrangement (U.S. Pat. No. 5,565,332), which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO/9852976 A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

An scFv can be prepared by the technique for producing a single chain unit (U.S. Pat. No. 4,946,778; Bird, Science 242:423-426 (1988), Huston et al., Proc. Natl. Acad. Sci. USA 85:5879-5883 (1988), Ward et al., Nature 341:544-546 (1989), and Nie et al., Antibody Therapeutics 3(1):18-62 (2020)). Single chain fusion peptides are produced by amino acid bridging the heavy and light chain fragments of the Fv region to form a single chain unit. Techniques for the assembly of functional Fv fragments in *E. coli* may also be used (Skerra et al., Science 240: 1038-1041 (1988)).

Examples of techniques that can be used to produce a single chain Fv (scFv) and an antibody include, for example, those described in U.S. Pat. Nos. 4,946,778 and 5,258,498, and Huston et al., Methods in Enzymology, 203:46-88 (1991), Shu et al., Proc. Natl. Acad. Sci. USA, 90:7995-7999 (1993) and Skerra et al., Science, 240:1038-1040 (1988). For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see Morrison, Science 229:1202 (1985); Oi et al., BioTechniques 4:214 (1986); Gillies et al., J. Immunol. Methods 125:191-202 (1989); Neuberger et al., Nature 312:604-608 (1984); Takeda et al., Nature 314:452-454 (1985); and U.S. Pat. Nos. 5,807,715, 4,816,567 and 4,816,397, which are incorporated herein by reference in their entireties.

In some embodiments, the antibody of the present invention is prepared by using hybridoma techniques. For preparing monoclonal antibodies using, for example, hybridoma methods, see, e.g., those described by Kohler and Milstein, Nature, 256:495 (1975). In the hybridoma method, a mouse, a hamster or other appropriate host animal, is typically immunized with an immunizing agent to elicit production of lymphocytes or production of antibodies that can specifically bind to the immunizing agent.

The immunizing agent will generally include a protein antigen, a fragment thereof or a fusion protein thereof. Generally, if a human cell is desired, a peripheral blood lymphocyte is used; if a cell of non-human mammalian origin is desired, a spleen lymphocyte or lymph node cell is used. In some embodiments, a spleen lymphocyte is used; the lymphocyte is then fused with an immortalized cell line using a suitable fusing agent, such as polyethylene glycol, to form a hybridoma cell (Goding, Monoclonal Antibodies: Principles and Practice, Academic Press, (1986) pp. 59-103). The immortalized cell line is generally a transformed mammalian cell, particularly a myeloma cell of rodent, bovine or human origin. Generally, rat or mouse myeloma cell lines are used. In some embodiments, a spleen lymphocyte and a mouse myeloma cell are used for fusion. The hybridoma cell may be cultured in a suitable culture medium, and in some embodiments, the culture medium contains one or more substances that inhibit the growth or survival of unfused immortalized cells. For example, if the parent cells lack hypoxanthine-guanine phosphoribosyltransferase (HGPRT or HPRT), the culture medium for the hybridoma generally contains hypoxanthine, aminopterin and thymine ("HAT medium"), which prevent the growth of HGPRT-deficient cells. In some embodiments, the binding specificity of the monoclonal antibody produced by the hybridoma cells is determined by immunoprecipitation or an *in vitro* binding assay, such as radioimmunoassay (RIA) or enzyme-linked immunosorbent assay (ELISA). Such techniques and assays are known in the art. The binding affinity of the monoclonal antibody can be determined, for example, by Scatchard analysis described by Munson and Pollard, Anal. Biochem., 107:220 (1980). Furthermore, in the therapeutic application of the monoclonal antibody, it is important to identify antibodies with high specificity and high binding affinity for the target antigen.

After the desired hybridoma cells are identified, the clones can be subcloned using dilution cloning procedures and grown by standard methods (see Goding, Monoclonal Antibodies: Principles and Practice, Academic Press, (1986) pp. 59-103). Suitable media for this purpose include, for example, Dulbecco's modified Eagle's Medium and RPMI-1640 medium.

In some embodiments, the monoclonal antibody secreted by the subclone can be isolated or purified by conventional techniques, such as protein A-sepharose, hydroxyapatite chromatography, gel electrophoresis, dialysis, or affinity chromatography.

The monoclonal antibody can also be prepared by recombinant DNA methods, such as those described in U.S. Pat. No. 4,816,567. DNA encoding the monoclonal antibody of the present invention can be isolated and sequenced using conventional methods (e.g., by using oligonucleotide probes that can specifically bind to genes of the heavy chain and the light chain of antibodies). DNA encoding the antibody of the present invention can also be synthesized by conventional methods based on antibody sequence design. The isolated or synthetic DNA is inserted into an expression vector, and then transfected into a host cell such as a Chinese hamster ovary (CHO) cell, a human embryonic kidney (HEK) 293 cell, a simian COS cell, a PER. NS0 cell, an SP2/0 cell, a YB2/0 cell, or a myeloma cell that does not otherwise produce immunoglobulins, thereby obtaining a synthetic monoclonal antibody in the recombinant host cell.

In some embodiments, the antibody or the antigen-binding fragment thereof of the present invention is prepared by hybridoma techniques: a protein formulation comprising CLDN18.2 is used to immunize a mouse; spleen lymphocytes of the immunized mouse are fused with myeloma cells; the hybridoma cells are screened through the CLDN18.2 protein; the positive hybridoma cells are subjected to dilution cloning and further subcloning; and the binding capacity of the hybridoma cell strains to the CLDN18.2 protein is identified again for producing the anti-CLDN18.2 antibody. In some embodiments, the mouse is a female Balb/c mouse aged 6-8 weeks.

The binding specificity of the antibody or the antigen-binding fragment of the present invention can be detected by an *in vitro* assay, such as co-immunoprecipitation, radioimmunoassay (RIA) or enzyme-linked immunosorbent assay (ELISA).

For certain use, including *in vivo* use of antibodies in humans and *in vitro* detection assays, in some embodiments, a chimeric antibody, a humanized antibody, or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see Morrison, Science, 229:1202 (1985), Oi et al., BioTechniques, 4:214 (1986), Gillies et al., J. Immunol. Methods, 125:191-202 (1989), and U.S. Patent Nos. 5,807,715, 4,816,567 and 4,816,397, which are incorporated herein by reference in their entireties.

Humanized antibodies can also be produced by transgenic mice that cannot express functional endogenous immunoglobulins but express human immunoglobulin genes. For example, human heavy and light chain immunoglobulin gene complexes can be introduced randomly or by homologous recombination into mouse embryonic stem cells. Alternatively, in addition to the human heavy and light chain genes, human variable, constant and diversity regions can be introduced into mouse embryonic stem cells. Mouse heavy and light chain immunoglobulin genes can be disabled by homologous recombination through introducing human immunoglobulin loci. In particular, homozygous deletion of the JH region can prevent the production of endogenous antibodies. The modified embryonic stem cells are expanded and microinjected into blastocysts to produce chimeric mice. The chimeric mice are then bred to produce homozygous progeny that express humanized antibodies. The transgenic mice are immunized by conventional methods with selected antigens, e.g., all or part of the desired polypeptide targets. Antigen-targeting monoclonal antibodies can be obtained from the immunized transgenic mice using conventional hybridoma techniques. The human immunoglobulin transgenes carried by the transgenic mice rearrange during B cell differentiation, resulting in class switching and somatic mutation. Accordingly, using this technique, IgG, IgA, IgM, and IgE antibodies that are useful for therapy can be produced.

In other embodiments, DNA encoding the desired monoclonal antibody can be readily isolated and sequenced using conventional methods (e.g., using oligonucleotide probes that can specifically bind to genes encoding the heavy and light chains of a murine antibody). Once isolated, the DNA can be inserted into an expression vector and then transfected into prokaryotic or eukaryotic host cells such as *E. coli* cells, simian COS cells, Chinese hamster ovary (CHO) cells, or myeloma cells that do not otherwise produce immunoglobulins. The isolated DNA (which may be synthetic as described herein) can be used to prepare sequences of the constant and variable regions of an antibody, as described in U.S. Pat. No. 5,658,570, which is incorporated herein by reference in its entirety. This method extracts RNA from the selected cells and converts it to cDNA, followed by amplification by PCR using Ig-specific primers. Suitable probes for this purpose are also described in U.S. Pat. No. 5,658,570.

In addition, using conventional recombinant DNA techniques, one or more CDRs of the antibody or the antigen-binding fragment of the present invention can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of a target antigen. One or more amino acid substitutions are made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

In addition, another efficient method for producing recombinant antibodies, which in particular is capable of producing primate antibodies comprising monkey variable region and human constant region sequences, is disclosed in Newman, Biotechnology 10:1455-1460 (1992), which is incorporated herein by reference in its entirety. Furthermore, this technique is also described in commonly assigned U.S. Pat. Nos. 5,658,570, 5,693,780 and 5,756,096, each of which is incorporated herein by reference in its entirety.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

In some embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, and the like. Commonly used mammalian host cells include 293 cells, Cos1 cells, Cos7 cells, CV1 cells, murine L cells, CHO cells, and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the original heavy chain variable region and light chain variable region. Alternatively, mutations can be introduced randomly along all or part of the coding sequence, for example by saturation mutagenesis, and the resulting mutants can be screened for the biological activity to identify mutants that retain the activity.

### Treatment Method

The present invention further provides a treatment method and use. In some embodiments, provided is a method for treating or ameliorating various types of cancers or tumors and other related diseases, wherein the method comprises administering to a patient in need thereof an effective dose of the anti-CLDN18.2 antibody or the antigen-binding fragment. In some embodiments, provided is use of the anti-CLDN18.2 antibody or the antigen-binding fragment in treating or ameliorating cancers or tumors and other related diseases. In some embodiments, provided is use of the anti-CLDN18.2 antibody or the antigen-binding fragment in the manufacture of a medicament for treating or ameliorating cancers or tumors and other related diseases.

The specific dosage and therapeutic regimen for any particular patient will depend upon a variety of factors including the particular antibody, the antigen-binding fragment or derivative thereof used, the age and body weight of the patient, general health condition, sex and diet, and the time of administration, frequency of excretion, drug combination, and the severity of the particular disease being treated. These factors are judged by medical caregivers included within the scope of those of ordinary skills in the art. The dosage will also depend on the individual patient to be treated, the route of administration, the type of the formulation, the nature of the compound used, the severity of the disease and the efficacy desired. The dosage employed can be determined by pharmacological and pharmacokinetic principles well known in the art. In some embodiments, the antibody of the present invention is administered to a patient at a dose of 0.01 mg/kg to 100 mg/kg of patient body weight per administration. In some embodiments, the administration is performed once every week, every 2 weeks, every 3 weeks, or every month.

The modes of administration for the antibody and the antigen-binding fragment thereof include, but are not limited to, intradermal, intramuscular, intraperitoneal, intravenous, subcutaneous, nasal and epidural injections and oral administration. The antibody, the antigen-binding fragment, or the composition can be administered by any convenient route, for example by infusion or bolus injection, or epithelial or mucocutaneous absorption (e.g., through oral mucosa, rectal, and intestinal mucosa), and can be co-administered with other biologically active agents. Thus, the pharmaceutical composition comprising the antibody or the antigen-binding fragment of the present invention can be administered orally, rectally, parenterally, intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous and intraarticular injections and infusions.

The mode of administration may be systemic or local.

The antibody, the antigen-binding fragment, or the pharmaceutical composition of the present invention may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. In some embodiments, when administering the protein (including the antibody) of the present invention, care must be taken to use materials that do not absorb the protein.

In some embodiments, the composition of the present invention comprises a nucleic acid or a polynucleotide encoding an antibody, which can be administered *in vivo* to facilitate expression of the encoded protein by constructing the nucleic acid or the polynucleotide as part of a suitable nucleic acid expression vector; and a part of the vector is administered to be intracellular by the following methods, for example, by using a retroviral vector (see U.S. Pat. No. 4,980,286), by direct injection, by using microprojectile bombardment (e.g., gene gun; Biolistic, Dupont), by coating with lipids or cell surface receptors or transfection reagents, or by ligation with homeobox-like peptides with known capability of entering the nucleus (see, e.g., Joliot et al., 1991, Proc. Natl. Acad. Sci. USA 88:1864-1868). Alternatively, the nucleic acid can be introduced into the cell and integrated into the host cell DNA for expression by homologous recombination. Methods for treating diseases comprising administering the antibody, the antigen-binding fragment or derivative thereof described herein are generally tested *in vitro,* followed by *in vivo* tests for desired therapeutic or prophylactic activities in an acceptable animal model, and finally administration in humans. Suitable animal models (including transgenic animals) are well known to those of ordinary skills in the art. For example, *in vitro* assays for demonstrating therapeutic use of the antibody or the antigen-binding fragment of the present invention include the effect of the antibody or the antigen-binding fragment on a cell line or a patient tissue sample. The effect of the antibody or the antigen-binding fragment on the cell line and/or the tissue sample can be detected using techniques known to those skilled in the art, such as the techniques disclosed elsewhere herein. In accordance with the teachings of the present invention, *in vitro* assays that can be used to determine whether to administer a specific antibody or antigen-binding fragment include *in vitro* cell culture experiments, wherein a patient tissue sample is cultured in a culture medium and is exposed to or otherwise administered a compound, and the effect of the compound on the tissue sample is observed.

Various known delivery systems can be used to administer the antibody of the present invention or the polynucleotide encoding the antibody of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors, or the like.

### Combination Therapy

In some embodiments, the anti-CLDN18.2 antibody or the antigen-binding fragment of the present invention can be administered in combination with other therapeutic or prophylactic regimens, including administration of one or more antibodies or antigen-binding fragments of the present invention together or in combination with one or more other therapeutic agents or methods. In some embodiments, other therapeutic regimens include, but are not limited to, radiation therapy, chemotherapy, hormone therapy, and the like. For combination therapy, the antibody may be administered simultaneously or separately with other therapeutic agents. When administered separately, the antibody of the present invention can be administered before or after administration of another additional therapeutic agent.

In some embodiments, the antibody of the present invention is administered in combination with a chemotherapeutic agent. In some embodiments, chemotherapeutic agents that can be administered with the antibody of the present invention include, but are not limited to, carboplatin (Paraplatin), cisplatin (platinol, platinol-AQ), cyclophosphamide (Endoxan, cyclophosphamide), docetaxel (Taxotere), doxorubicin (adriamycin), erlotinib (Tarceva), etoposide (Vepesid), fluorouracil (5-FU), gemcitabine (Gemzar), imatinib mesylate (Gleevec), irinotecan (irinotecan), methotrexate (diazo tablet, methotrexate sodium, methotrexate), paclitaxel (Taxol, Abraxane), sorafenib (Nexavar), sunitinib (Sutent), topotecan (Hycamtin), vincristine (Oncovin, Vincasar PFS), and vinblastine (Velban).

In some embodiments, the antibody of the present invention is administered in combination with a cytokine, a chemokine, or a co-stimulatory molecule. In some embodiments, cytokines, chemokines or co-stimulatory molecules that may be administered with the antibody of the present invention include, but are not limited to, CCR7, CCL19, CCL21, CCL2, CCL3, CCL5, CCL16, CXCR4, CXCR7, CXCL12, interleukins (e.g., IL-1-IL17), interferons (e.g., IFNα1, IFNα8, IFNα10, IFNα13, IFNα14, IFNα16, IFNα17, IFNα21, IFNβ1, IFNW, IFNE1, and IFNK), hematopoietic growth factors, TGFs (e.g., TGF-α, TGF-β, and other members of the TGF family), 4-1BB, 4-1BB-L, CD137, CD137L, CTLA-4GITR, GITRL, Fas, Fas-L, TNFR1, TRAIL-R1, TRAIL-R2, p75NGF-R, DR6, RANK, EDAR1, XEDAR, Fn114, Troy/Trade, TAJ, TNFRII, HVEM, CD27, CD30, CD40, 4-1BB, OX40, GITR, GITRL, TACI, BAFF-R, BCMA, RELT, CD95(Fas/APO-1), glucocorticoid-induced TNFR-related protein, TNF receptor-related apoptosis-mediating protein (TRAMP), death receptor-6 (DR6), and the like.

In some embodiments, the antibody of the present invention is administered in combination with a immunotherapeutic agent. In some embodiments, immunotherapeutic agents that may be administered with the antibody of the present invention include, but are not limited to, abagovomab (CA-125), abciximab (CD41), adecatumumab (EpCAM), afutuzumab (CD20), alacizumab pegol (VEGFR2), altumomab pentetate (CEA), amatuximab (MORAb-009), anatumomab mafenatox (TAG-72), apolizumab (HLA-DR), arcitumomab (CEA), bavituximab (phosphatidylserine), bectumomab (CD22), belimumab (BAFF), bevacizumab (VEGF-A), bivatuzumab (CD44 v6), blinatumomab (CD19), brentuximab vedotin (CD30 TNFRSF8), cantuzumab mertansine (mucin CanAg), cantuzumabravtansine (MUC1), capromab pendetide (prostate cancer cell), carlumab (CNTO888), catumaxomab (EpCAM, CD3), cetuximab (EGFR), citatuzumab bogatox (EpCAM), cixutumumab (IGF-1 receptor), clenoliximab (tight-junction protein), clivatuzumab tetraxetan (MUC1), conatumumab (TRAII,-R2), dacetuzumab (CD40), dalotuzumab (insulin-like growth factor I receptor), denosumab (RANKL), detumomab (B lymphoma cell), dacetuzumab (DR5), ecromeximab (GD3 ganglioside), efungumab (EpCAM), elotuzumab (SLAMF7), enavatuzumab (PDL192), ensituximab (NPC-1C), epratuzumab (CD22), ertumaxomab (HER2/neu, CD3), etaracizumab (integrin αvβ3), farletuzumab (folate receptor 1), FBTA05 (CD20), ficlatuzumab (SCH 900105), figitumumab (IGF-1 receptor), flanvotumab (glycoprotein 75), fresolimumab (TGF-β), galiximab (CD80), ganitumab (IGF-I), gemtuzumab (CD33), Gevokizumab (IL-1β), girentuximab (carbonic anhydrase 9(CA-IX)), ibritumomab tiuxetan (CD20), icrucumab (VEGFR-1), igovomab (CA-125), indatuximabravtansine (SDC1), intetumumab (CD51), inotuzumab ozogamicin (CD22), ipilimumab (CD152), iratumumab (CD30), labetuzumab (CEA), lexatumumab (TRAII,-R2), libivirumab (hepatitis B surface antigen), lintuzumab (CD33), lorvotuzumab mertansine (CD56), lucatumumab (CD40), lumiliximab (CD23), mapatumumab (TRAIL-R1), matuzumab (EGFR), mepolizumab (IL-5), milatuzumab (CD74), mitumomab (GD3 ganglioside), mogamulizumab (CCR4), moxetumomab pasudotox (CD22), nacolomab tafenatox (C242 antigen), naptumomab estafenatox (5T4), narnatumab (RON), necitumumab (EGFR), nimotuzumab (EGFR), nivolumab (IgG4), ofatumumab (CD20), olaratumab (PDGF-Rα), onartuzumab (human discrete factor receptor kinase), oportuzumab monatox (EpCAM), oregovomab (CA-125), oxelumab (OX-40), pemtumomab (EGFR), patritumab (HER3), pemtumomab (MUC1), pertuzumab (HER2/neu), pintumomab (adenocarcinoma antigen), pritumumab (vimentin), racotumomab (N-glycolylneuraminic acid), radretumab (extra domain B of fibronectin), rafivirumab (rabies virus glycoprotein), ramucirumab (VEGFR2), rilotumumab (HGF), rituximab (CD20), robatumumab (IGF-1 receptor), samalizumab (CD200), sibrotuzumab (FAP), Siltuximab (IL-6), tabalumab (BAFF), tacatuzumab tetraxetan (α-fetoprotein), taplitumomab paptox (CD19), tenatumomab (tenascin C), teprotumumab (CD221), ticilimumab (CTLA-4), tigatuzumab (TRAIL-R2), TNX-650 (IL-13), tositumomab (CD20), trastuzumab (HER2/neu), TRBS07 (GD2), tremelimumab (CTLA-4), tucotuzumab celmoleukin (EpCAM), ublituximab (MS4A1), urelumab (4-1BB), and volociximab (integrin α5β1).

### Diagnostic Methods

Expression of CLDN18.2 is observed in certain cancer or tumor samples, and patients with cells positively expressing CLDN18.2 respond to treatment with the anti-CLDN18.2 antibody of the present invention. Thus, the antibody or the antigen-binding fragment of the present invention can also be used for diagnosis and prognosis.

A sample comprising cells may be obtained from a patient, which may be a cancer patient or a patient to be diagnosed. The cells are cells of a tumor tissue or tumor mass, a blood sample, a urine sample, or any sample from the patient. After an optional pretreatment of the sample, the sample can be incubated with the antibody of the present invention in a condition that allows the antibody to interact with CLDN18.2 protein that may be present in the sample. By methods such as ELISA, the presence and the expression level of CLDN18.2 protein in the sample can be detected using the anti-CLDN18.2 antibody.

The presence of CLDN18.2 protein (at any amount or concentration) in a sample can be used to diagnose a cancer, which may be an indication that the patient is eligible for an antibody therapy, or an indication that the patient has (or has not) responded to a cancer therapy. For prognostic methods, one, two or more tests may be performed at a particular stage after the beginning of a cancer therapy to indicate the progress of the therapy.

### Composition

The present invention further provides a pharmaceutical composition. Such a composition comprises an effective dose of the anti-CLDN18.2 antibody (or the antigen-binding fragment) and a pharmaceutically acceptable carrier. In some embodiments, the pharmaceutical composition comprises 0.1%-90% of the anti-CLDN18.2 antibody or the antigen-binding fragment. In some embodiments, the pharmaceutical composition further comprises an anti-cancer agent (e.g., an immune checkpoint inhibitor).

In one specific embodiment, the term "pharmaceutically acceptable" refers to materials listed in pharmacopoeia for use in animals, in particular in humans. In addition, the "pharmaceutically acceptable carrier" will generally be any type of non-toxic solid, semi-solid or liquid filler, diluent, encapsulating material, or formulation auxiliary.

The term "carrier" refers to a diluent, adjuvant, excipient or carrier that can be administered to a patient together with the active ingredient. Such pharmaceutical carriers can be sterile liquids, such as water and oils, including oils originated from petroleum, animal, plant or synthesis, such as peanut oil, soybean oil, mineral oil, sesame oil, and the like. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol, and the like. If desired, the composition may also comprise a small amount of a wetting agent, an emulsifier, or a pH buffering agent such as acetates, citrates or phosphates. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or antigen-binding fragment, preferably in purified form, together with an appropriate amount of a carrier, to provide a form suitable for administration to a patient. The formulation should be suitable for the mode of administration. The parental formulation may be encapsulated in an ampoule bottle, a disposable syringe, or a multi-dose vial made of glass or plastic.

In some embodiments, the composition is formulated into a pharmaceutical composition suitable for intravenous injection into a human body according to conventional steps. A composition for intravenous administration is a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

The compound of the present invention may be formulated in a neutral or salt form. Pharmaceutically acceptable salts include salts formed with anions derived from, e.g., hydrochloric acid, phosphoric acid, acetic acid, oxalic acid and tartaric acid, and salts formed with cations derived from, e.g., sodium, potassium, ammonium, calcium, iron hydroxide, isopropylamine, triethylamine, 2-ethylaminoethanol, histidine, and procaine.

"About" refers to a general error range for corresponding values as readily understood by those skilled in the relevant art. In some embodiments, "about" mentioned herein refers to the values described and ranges thereof of ±10%, ±5%, or ±1%.

"EC₅₀", i.e., concentration for 50% of maximal effect, refers to a concentration that causes 50% of maximal effect.

"IC ₅₀", i.e., half maximal inhibitory concentration, refers to a half inhibitory concentration of an antagonist.

### Example 1: Preparation of Immune Antigens

### 1. Construction of CHO-CLDN18.2 stable cell line

The full-length amino acid sequence of human CLDN18.2 (from NCBI, NM_001002026.3) is as follows:

"MAVTACQGLGFVVSLIGIAGIIAATCMDQWSTQDLYNNPVTAVFNYQGLWRSCVRESSGF
TECRGYFTLLGLPAMLQAVRALMIVGIVLGAIGLLVSIFALKCIRIGSMEDSAKANMTLTSG
IMFIVSGLCAIAGVSVFANMLVTNFWMSTANMYTGMGGMVQTVQTRYTFGAALFVGWV
AGGLTLIGGVMMCIACRGLAPEETNYKAVSYHASGHSVAYKPGGFKASTGFGSNTKNKKI
YDGGARTEDEVQSYPSKHDYV" (SEQ ID NO: 93).

The corresponding nucleic acid sequence is as follows:

"atggccgtgactgcctgtcagggcttggggttcgtggtttcactgattgggattgcgggcatcattgctgccacctgcatggaccagtggag
cacccaagacttgtacaacaaccccgtaacagctgttttcaactaccaggggctgtggcgctcctgtgtccgagagagctctggcttcaccga
gtgccggggctacttcaccctgctggggctgccagccatgctgcaggcagtgcgagccctgatgatcgtaggcatcgtcctgggtgccattg
gcctcctggtatccatctttgccctgaaatgcatccgcattggcagcatggaggactctgccaaagccaacatgacactgacctccgggatca
tgttcattgtctcaggtctttgtgcaattgctggagtgtctgtgtttgccaacatgctggtgactaacttctggatgtccacagctaacatgtacacc
ggcatgggtgggatggtgcagactgttcagaccaggtacacatttggtgcggctctgttcgtgggctgggtcgctggaggcctcacactaatt
gggggtgtgatgatgtgcatcgcctgccggggcctggcaccagaagaaaccaactacaaagccgtttcttatcatgcctcaggccacagtgt
tgcctacaagcctggaggcttcaaggccagcactggctttgggtccaacaccaaaaacaagaagatatacgatggaggtgcccgcacaga
ggacgaggtacaatcttatccttccaagcacgactatgtg" (SEQ ID NO: 94).

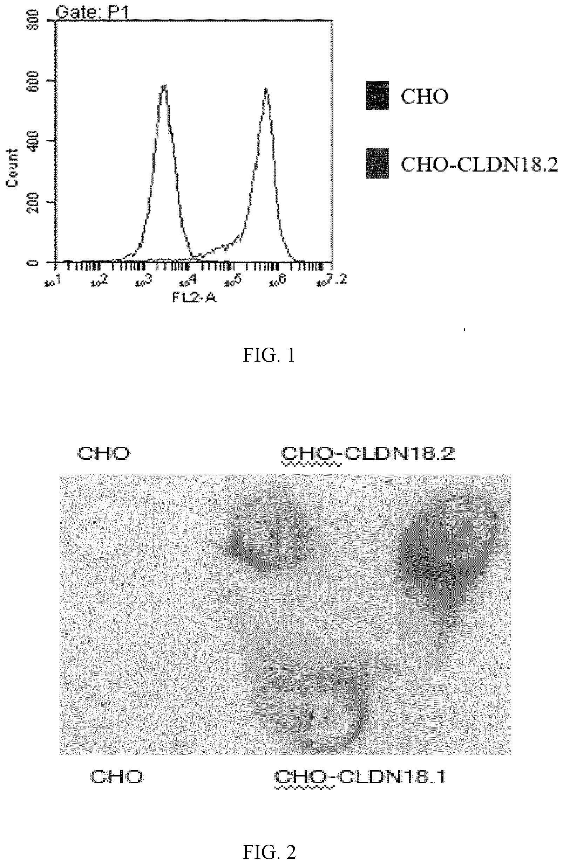

The nucleic acid sequence corresponding to human CLDN18.2 described above was synthesized, and sequences of enzyme digestion sites HindIII and EcoRI were added at both ends of the sequence. The sequence was then constructed into the pcDNA3.1 expression vector (Invitrogen, Cat. No. V79020). The expression vector was transfected into CHO cells (Life technologies, Cat. No. A13696-01) by electroporation. The electroporation conditions were as follows: voltage 300 V, time 17 ms, and 4 mm electroporation cuvette. After 48 h, 50 µM MSX (methionine iminosulfone) was added as a screening pressure, and positive cells were selected after 2 weeks. High-expression cell lines were selected by FACS. The cells were collected and washed once with PBS (phosphate-buffered saline, 1 L of PB S containing 8.0 g of NaCl, 0.9 g of Na₂HPO₄, 0.156 g of KH₂PO₄, and 0.125 g of KCl; pH 7.2-7.4). 3 µg/mL anti-CLDN18.2 antibody (IMAB362, the sequence of which is the same as that of the antibody expressed by hybridoma cells 175D10 in Patent No. US20090169547, prepared in the same manner as in Example 5 and Example 6 of the present application) was added, and the mixture was incubated at 4 °C for 1 h and washed twice with PBS. 100 µL of 1:500 diluted goat anti-human IgG-Fc PE fluorescent secondary antibody (Cat. No. 12-4998-82, eBioscience) was added, and the mixture was incubated at 4 °C for 1 h, washed twice with PBS, and analyzed by a C6 flow cytometer (BD, model: C6 flow cytometer). The cells obtained were named CHO-CLDN18.2 cells, and the FACS results are shown in FIG. 1.

### 2. Construction of CHO-CLDN18.1 stable cell line

The full-length amino acid sequence of human CLDN18.1 (from NCBI, NM_016369.4) is as follows:

"MSTTTCQVVAFLLSILGLAGCIAATGMDMWSTQDLYDNPVTSVFQYEGLWRSCVRQSSG
FTECRPYFTILGLPAMLQAVRALMIVGIVLGAIGLLVSIFALKCIRIGSMEDSAKANMTLTS
GIMFIVSGLCAIAGVSVFANMLVTNFWMSTANMYTGMGGMVQTVQTRYTFGAALFVGW
VAGGLTLIGGVMMCIACRGLAPEETNYKAVSYHASGHSVAYKPGGFKASTGFGSNTKNK
KIYDGGARTEDEVQSYPSKHDYV" (SEQ ID NO: 95).

The corresponding nucleic acid sequence is as follows:

"atgtccaccaccacatgccaagtggtggcgttcctcctgtccatcctggggctggccggctgcatcgcggccaccgggatggacatgtgg

agcacccaggacctgtacgacaaccccgtcacctccgtgttccagtacgaagggctctggaggagctgcgtgaggcagagttcaggcttca

ccgaatgcaggccctatttcaccatcctgggacttccagccatgctgcaggcagtgcgagccctgatgatcgtaggcatcgtcctgggtgcc

attggcctcctggtatccatctttgccctgaaatgcatccgcattggcagcatggaggactctgccaaagccaacatgacactgacctccggg

atcatgttcattgtctcaggtctttgtgcaattgctggagtgtctgtgtttgccaacatgctggtgactaacttctggatgtccacagctaacatgta

caccggcatgggtgggatggtgcagactgttcagaccaggtacacatttggtgcggctctgttcgtgggctgggtcgctggaggcctcacac

taattgggggtgtgatgatgtgcatcgcctgccggggcctggcaccagaagaaaccaactacaaagccgtttcttatcatgcctcaggccaca

gtgttgcctacaagcctggaggcttcaaggccagcactggctttgggtccaacaccaaaaacaagaagatatacgatggaggtgcccgcac

agaggacgaggtacaatcttatccttccaagcacgactatgtg" (SEQ ID NO: 96).

The nucleic acid sequence corresponding to human CLDN18.1 described above was synthesized, and sequences of enzyme digestion sites HindIII and EcoRI were added at both ends of the sequence. The sequence was then constructed into the pcDNA3.1 expression vector. The expression vector was transfected into CHO cells by electroporation. The electroporation conditions were as follows: voltage 300 V, time 17 ms, and 4 mm electroporation cuvette. After 48 h, 50 µM MSX was added as a screening pressure, and positive cells were selected after 2 weeks. DotBlot hybridization was used for detection. The cells were collected, and the cell lysate supernatant was extracted, with blank CHO cell lysate supernatant as negative control and CHO-CLDN18.2 cell lysate supernatant as positive control. 20 µL of cell lysate was taken, spotted on an activated PVDF membrane (millipore), and left to stand for 3-5 min so that the membrane fully adsorbed the proteins. The membrane was soaked in 5% skimmed milk powder, blocked at 37 °C for 2 h, and washed thrice with PBST (PBS + 0.05% Tween). Then the membrane was soaked in rabbit anti-human Claudin18 antibody (Cat. No. ab230224, abcam, the antibody can recognize the same intracellular segment of CLDN18.1 and CLDN18.2, so CHO-CLDN18.2 cells can be used as positive control), incubated for 2 h at room temperature, and washed thrice with PBST. Then the membrane was soaked in goat anti-rabbit HRP antibody (Cat. No. ab6721, abcam), incubated for 2 h at room temperature, and washed thrice with PBST. Then DAB (Cat. No. AR1000, Boster Biological Technology) was used for color development. The DotBlot results are shown in FIG. 2. The cells obtained were named CHO-CLDN18.1 cells.

### Example 2: Preparation of Anti-Human CLDN18.2 Hybridoma Antibodies

### 1. Animal immunization

The animals for immunization were 9 female Balb/c mice at 6-8 weeks of age (Guangdong Medical Laboratory Animal Center, animal production license number: SCXK (Guangdong) 2013-0002) housed in an SPF environment. The purchased mice were housed for one week, after which they were immunized.

The immunization regimen was as follows:
10 million CHO-CLDN18.2 cells were injected intraperitoneally. The immune cycle was once every 2 weeks, and blood was taken from the tail vein on day 10 after the third immunization. The titer of the anti-human CLDN18.2 antibody in mouse plasma was determined by adopting a flow cytometry binding method so as to monitor the immune response degree in mice. If the titer result (1:10000 dilution) did not meet the requirement, the immunization was continued. A total of 5 rounds of immunization were carried out in the experiment. The mice were immunized via tail vein with a booster dose of 1 million CHO-CLDN18.2 cells 3 days prior to the fusion of splenocytes and myeloma cells.

### 2. Determination of titer of anti-human CLDN18.2 antibody in mouse plasma by flow cytometry

Flow cytometry was used. Human gastric cancer cells KATOIII (Cell Bank of Chinese Academy of Sciences, Shanghai) were collected, washed once with PBS, and plated on a 96-well V-bottom plate (Cat. No. 3897, Costar) at 0.3 million cells/50 µL. The cells were centrifuged at 2000 rpm for 5 min, and the supernatant was discarded. Tail vein blood of the immunized mice described above was extracted and diluted to 1:10000 with PBS. The cells described above were resuspended at 100 µL/well and mixed well. The mixture was incubated at 4 °C for 1 h and washed twice with PBS. Goat anti-mouse IgG-A647 fluorescent secondary antibody (Cat. No. bs-0296G-AF647; Bioss) (1: 500 dilution) was added at 100 µL/well; the mixture was incubated at 4 °C for 1 h, the plate was washed twice, and the titer was detected with a CytoFLEX flow cytometer (model: AOO-1-1102, Beckman). The detection results of the titer prior to the fusion are as follows:

**Table 1. Detection results of titer of tail vein blood of mice (1:10000)**

| Sample | Binding positive rate (%) | Fluorescence value |
|---|---|---|
| Balb/c negative serum | 0.15% | 1397.8 |
| Balb/c-1 | 28.28% | 7555.7 |
| Balb/c-2 | 22.40% | 4569.2 |
| Balb/c-3 | 28.14% | 15757.7 |
| Balb/c-4 | 37.84% | 16446.6 |
| Balb/c-5 | 35.18% | 15718.9 |
| Balb/c-6 | 35.68% | 14114.7 |
| Balb/c-7 | 34.00% | 9147.3 |
| Balb/c-8 | 39.76% | 14611.5 |
| Balb/c-9 | 39.06% | 16824.5 |

From the detection results of titer described above, it can be seen that after 5 rounds of immunization, all mice developed anti-CLDN18.2 antibodies; except that the binding activity of the antibodies in the serum of 3 mice, namely Balb/c-1, Balb/c-2, and Balb/c-7, was slightly poor, the binding fluorescence values of the antibodies in the serum of the other mice were all about 15000, which was more than 10 times that of the negative serum; therefore, the fusion could be performed.

### 3. Splenocyte fusion

One day before the fusion, blank mouse peritoneal macrophages were taken as feeder cells, resuspended in an RPMI1640 medium containing 10% FBS, 1× HAT, and 1× diabody (the diabody was penicillin and streptomycin), and plated on 96-well cell culture plates at 100 µL/well. On the day of the fusion, splenocytes of Balb/c-3, Balb/c-4, and Balb/c-9 mice were aseptically extracted, and myeloma cells SP2/0 (Cell Bank of Chinese Academy of Sciences, Shanghai) and the splenocytes were fused at a ratio of 1:5 using polyethylene glycol (PEG solution 50% (W/V), Cat. No. P7181, Sigma) to generate hybridoma cells. The fused cells were resuspended in 100 mL of RPMI1640 complete medium containing 10% FBS, 1× HAT, and 1× diabody, and plated on the 96-well plates containing 100 µL/well feeder cells. The amount of the fused cells was also 100 µL/well, for a total of ten 96-well plates. The cells were incubated at 37 °C with 5% CO₂. A half medium exchange was carried out every 3-4 days, that is, 100 µL/well of the culture medium was removed, and then 100 µL/well of fresh HAT complete culture medium was supplemented. Flow cytometry was performed on days 7-10 according to the size of the clones formed.

### 4. Screening of hybridoma cells

CLDN18.2 positive cells (KATOIII or CHO-CLDN18.2) labeled with fluorescent dye CFSE (Cat. No. C34554, Invitrogen) and CHO-CLDN18.1 cells that were not labeled were mixed at a ratio of 1:1 (0.1 million cells each), 100 µL of the supernatant of the fused cells to be assayed were added, and the mixture was incubated at 4 °C for 1 h and washed twice with PBS. Then 100 µL of 1:500 diluted goat anti-mouse IgG-A647 fluorescent secondary antibody (Cat. No. bs-0296G-AF647; Bioss) was added. The mixture was incubated at 4 °C for 1 h, washed twice with PBS, and analyzed with a CytoFLEX flow cytometer (model: AOO-1-1102, Beckman). A scatter diagram was drawn using the CFSE-labeled fluorescence intensity (FITC channel) as the abscissa and the goat anti-mouse IgG-A647-labeled fluorescence intensity (APC channel) as the ordinate, and the binding of the cells in the supernatant to be assayed was analyzed.

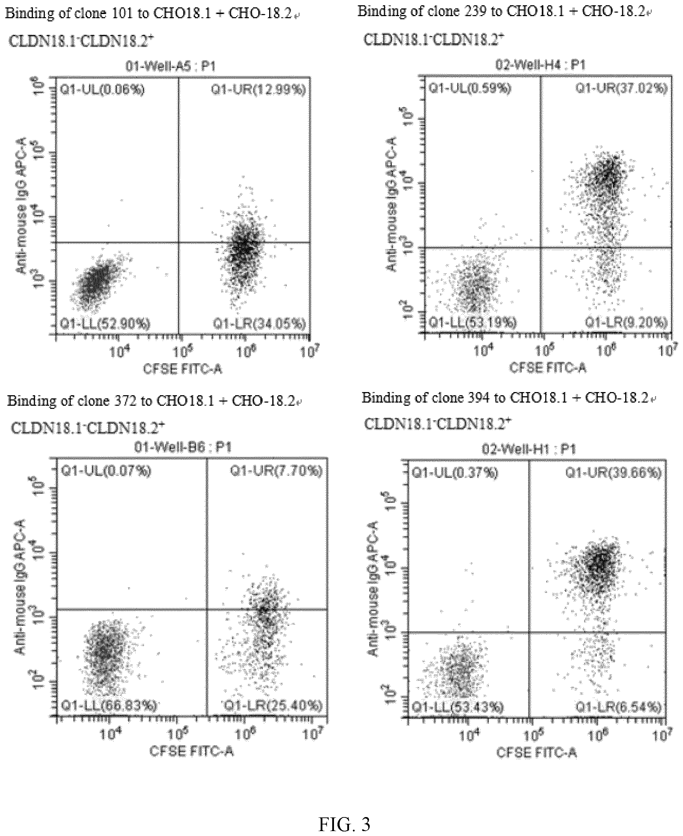

The detection results show that clones 101, 239, 372, and 394 were able to specifically bind to CLDN18.2 and not to CLDN18.1 (the results are shown in FIG. 3).

### Example 3: Sequence Acquisition of Hybridoma Positive Clones

Hybridoma cells 101, 239, 372, and 394 in the logarithmic phase were harvested, and the total cellular RNA was extracted using the EasyPure RNA Kit (Cat. No. ER101; TRANSGEN). The obtained RNA was reverse-transcribed into cDNA using the SuperScript IV First-Strand Synthesis System kit (Cat. No. 18091200, Invitrogen). The heavy and light chain variable region DNA sequences were amplified by PCR using the cDNA as a template and linked to a T-vector (Cat. No. CB111, TRANSGEN) for sequencing.

The primers for amplification of the heavy chain variable region were as follows (synthesized by Sangon Biotech (Shanghai) Co., Ltd.):
VhRevU: GAG GTS MAR CTG CAG SAG TCW GG (SEQ ID NO: 97) (M and R represent degenerate bases); and
VhForU: GAC AGT GGA TAR ACM GAT GG (SEQ ID NO: 98).

The primers for amplification of the light chain variable region were as follows (synthesized by Sangon Biotech (Shanghai) Co., Ltd.):
VkRev1: GAG GTS MAR CTG CAG SAG TCW GG (SEQ ID NO: 99);
VkRev2: GAT ATT GTG ATG ACG CAG GCT (SEQ ID NO: 100);
VkRev3: GAT ATT GTG ATAACC CAG (SEQ ID NO: 101);
VkRev4: GAC ATT GTG CTG ACC CAA TCT (SEQ ID NO: 102);
VkRev5: GAC ATT GTG ATG ACC CAG TCT (SEQ ID NO: 103);
VkRev6: GAT ATT GTG CTA ACT CAG TCT (SEQ ID NO: 104);
VkRev7: GAT ATC CAG ATG ACA CAG ACT (SEQ ID NO: 105);
VkRev8: GAC ATC CAG CTG ACT CAG TCT (SEQ ID NO: 106);
VkRev9: CAA ATT GTT CTC ACC CAG TCT (SEQ ID NO: 107); and
VkForU: GGA TAC AGT TGG TGC AGC ATC (SEQ ID NO: 108).

The obtained sequences of the heavy and light chain variable regions were as follows (CDR1-3 was underlined sequentially):

### ➢ Antibody 101

1) 101VH-1 heavy chain variable region amino acid sequence (SEQ ID NO: 1): 
EVQLQESGPELVKPGASVKISCKASGYSFTSYYIHWVKQRPGQGLEWIGWIYPGSGNTKY

NEKFKGKATLTADTSSSTAYMQLSSLTSEDSAVYYCARGMDYGNYYLDYWGQGTTLTVSS;
101VH-1 heavy chain variable region gene sequence (SEQ ID NO: 2): 
GAGGTGCAGCTGCAGGAGTCTGGACCTGAGCTGGTGAAGCCTGGGGCTTCAGTGAAG
ATATCCTGCAAGGCTTCTGGCTACAGCTTCACAAGCTACTATATACACTGGGTGAAGCA
GAGGCCTGGACAGGGACTTGAGTGGATTGGATGGATTTATCCTGGAAGTGGTAATACT
AAGTACAATGAGAAGTTCAAGGGCAAGGCCACACTGACGGCAGACACATCCTCCAGC
ACTGCCTACATGCAGCTCAGCAGCCTAACATCTGAGGACTCTGCGGTCTATTACTGTGC
AAGGGGGATGGACTATGGTAACTACTACCTTGACTACTGGGGCCAAGGCACCACTCTC
ACAGTCTCCTCA.
2) 101VL-1 light chain variable region amino acid sequence (SEQ ID NO: 3): 
DIVMTQSHKFMSTSVGDRVSITCKASQDVGTAVAWYQQKPGQSPKLLIYWASTRHTGVP
DRFTGSGSGTDFTLTISNVQSEDLADYFCQQYSSYLTFGAGTKLELK;
101VL-1 light chain variable region gene sequence (SEQ ID NO: 4): 
GACATTGTGATGACCCAGTCTCACAAATTCATGTCCACATCAGTAGGAGACAGGGTCA
GCATCACCTGCAAGGCCAGTCAGGATGTGGGTACTGCTGTAGCCTGGTATCAACAGAA
ACCAGGGCAATCTCCTAAACTACTGATTTACTGGGCATCCACCCGGCACACTGGAGTC
CCTGATCGCTTCACAGGCAGTGGATCTGGGACAGATTTCACTCTCACCATTAGCAATGT
GCAGTCTGAAGACTTGGCAGATTATTTCTGTCAGCAATATAGCAGCTATCTCACGTTCG
GTGCTGGGACCAAGCTGGAGCTGAAA.
3) 101VL-2 light chain variable region amino acid sequence (SEQ ID NO: 5): 
DIQLTQSPSSLSASLGDTITITCHASQNINVWLSWYQQKPGNIPKLLIYKASNLHTGVPSRF
SGSGSGTGFTLTISSLQPEDIATYYCQQGQSYPYTFGGGTKLEIK;
101VL-2 light chain variable region gene sequence (SEQ ID NO: 6):

GACATCCAGCTGACTCAGTCTCCATCCAGTCTGTCTGCATCCCTTGGAGACACAATTAC

CATCACTTGCCATGCCAGTCAGAACATTAATGTTTGGTTAAGCTGGTACCAGCAGAAAC

CAGGAAATATTCCTAAACTATTGATCTATAAGGCTTCCAACTTGCACACAGGCGTCCCA

TCAAGGTTTAGTGGCAGTGGATCTGGAACAGGTTTCACATTAACCATCAGCAGCCTGC

AGCCTGAAGACATTGCCACTTACTACTGTCAACAGGGTCAAAGTTATCCGTACACGTT

CGGAGGGGGGACCAAGCTGGAAATAAAA.

### ➢ Antibody 239

1) 239VH-2 heavy chain variable region amino acid sequence (SEQ ID NO: 7): 
EVKLQESGGGLVKPGGSLKVSCAASGFTFSDYGMHWVRQAPEKGLEWVAYISRGRSTTY
STDTVKGRFTISRDSAKHTLFLQMTSLRSEDTAMYYCARGSYYGNAMDYWGQGTSVTV
SS;
239VH-2 heavy chain variable region gene sequence (SEQ ID NO: 8): 
GAGGTCAAGCTGCAGGAGTCTGGGGGAGGCTTAGTGAAGCCTGGAGGGTCCCTGAAA
GTCTCCTGTGCAGCCTCTGGATTCACTTTCAGTGACTATGGAATGCACTGGGTTCGTCA
GGCTCCAGAGAAGGGGCTGGAGTGGGTTGCATACATTAGTAGGGGCAGAAGTACCAC
CTACTCTACAGACACAGTGAAGGGCCGATTCACCATCTCCAGAGACAGTGCCAAACAC
ACCCTGTTCCTGCAAATGACCAGTCTGAGGTCTGAGGACACGGCCATGTATTACTGTG
CAAGGGGAAGTTACTACGGTAATGCTATGGACTACTGGGGTCAAGGAACCTCAGTCAC
CGTCTCCTCA.
2) 239VH-9 heavy chain variable region amino acid sequence (SEQ ID NO: 9): 
EVKLQQSVAELVRPGASVKLSCTASGFNIKNTYIYWVKQRPEQGLEWIGRIDPANGNTKY
APKFQGKATITADTSSNTAYLQLSSLTSEDTAIYYCARSPAYYINYYAMDYWGQGTSVTVS
S;
239VH-9 heavy chain variable region gene sequence (SEQ ID NO: 10): 
GAGGTCAAGCTGCAGCAGTCTGTGGCAGAGCTTGTGAGGCCAGGGGCCTCAGTCAAG
TTGTCCTGCACAGCTTCTGGCTTCAACATTAAAAACACCTATATATACTGGGTGAAGCA
GAGGCCTGAACAGGGCCTGGAGTGGATTGGAAGGATTGATCCTGCGAATGGTAATACT
AAATATGCCCCGAAGTTCCAGGGCAAGGCCACTATAACTGCAGACACATCCTCCAACA
CAGCCTACCTGCAGCTCAGCAGCCTGACATCTGAGGACACTGCCATCTATTACTGTGCT
AGATCGCCGGCCTACTATATTAACTACTATGCTATGGACTACTGGGGTCAAGGAACCTC
AGTCACCGTCTCCTCA.
3) 239VL-1 light chain variable region amino acid sequence (SEQ ID NO: 11): 
QIVLTQSPAIMSASPGEKVTMTCSASSSVSYMHWYQQKSGTSPKRWIYDTSKLASGVPAR

FSGSGSGTSYSLTISSMEAEDAATYYCQQWSSNPFTFGSGTKLEIK;
239VL-1 light chain variable region gene sequence (SEQ ID NO: 12): 
CAAATTGTTCTCACCCAGTCTCCAGCAATCATGTCTGCATCTCCAGGGGAGAAGGTCA
CCATGACCTGCAGTGCCAGCTCAAGTGTAAGTTACATGCACTGGTACCAGCAGAAGTC
AGGCACCTCCCCCAAAAGATGGATTTATGACACATCCAAACTGGCTTCTGGAGTCCCT
GCTCGCTTCAGTGGCAGTGGGTCTGGGACCTCTTACTCTCTCACAATCAGCAGCATGG
AGGCTGAAGATGCTGCCACTTATTACTGCCAGCAGTGGAGTAGTAATCCATTCACGTTC
GGCTCGGGGACAAAGTTGGAAATAAAA.
4) 239VL-4 light chain variable region amino acid sequence (SEQ ID NO: 13): 
DIVMTQSQKFMSTSVGDRVSVTCKASQNVGTNVAWYQLKPGQSPKPLIYSTSYRYSGVP
DRFTGSGSGTDFTLTISNVQSEDLAEYFCQQYNSYPLTFGAGTKLELT;
239VL-4 light chain variable region gene sequence (SEQ ID NO: 14): 
GACATTGTGATGACCCAGTCTCAGAAATTCATGTCCACATCAGTTGGAGACAGGGTCA
GCGTCACCTGCAAGGCCAGTCAGAATGTGGGTACTAATGTTGCCTGGTATCAACTGAA
 
ACCAGGGCAATCTCCTAAACCACTGATTTACTCGACATCCTACCGGTACAGTGGAGTCC
CTGATCGCTTCACAGGCAGTGGATCTGGGACAGATTTCACTCTCACCATCAGCAATGTG
CAGTCTGAAGACTTGGCAGAGTATTTCTGTCAGCAATATAACAGCTATCCGCTCACGTT
CGGTGCTGGGACCAAGCTGGAGCTGACA.
5) 239VL-6 light chain variable region amino acid sequence (SEQ ID NO: 15): 
DIVMTQSPSSLTVTAGERVTMSCKSSQSLLNSGNQRNYLTWYQQKPGQPPKLLIYWASTR
ESGVPDRFTGSGSGTDFTLTISSVQAEDLAVYFCQSAYSYPFTFGSGTKLEIK;
239VL-6 light chain variable region gene sequence (SEQ ID NO: 16):

GACATTGTGATGACCCAGTCTCCATCCTCCCTGACTGTGACAGCAGGAGAGAGGGTCA
CTATGAGCTGCAAGTCCAGTCAGAGTCTGTTAAACAGTGGAAATCAAAGGAACTACTT
GACCTGGTACCAGCAGAAACCAGGGCAGCCTCCTAAACTGTTGATCTACTGGGCATCC
ACTAGGGAATCTGGGGTCCCTGATCGCTTCACAGGCAGTGGATCTGGAACAGATTTCA
CTCTCACCATCAGCAGTGTGCAGGCTGAAGACCTGGCAGTCTATTTCTGTCAGAGTGC
TTATAGTTATCCATTCACGTTCGGCTCGGGGACAAAGTTGGAAATCAAA.

### ➢ Antibody 372

1) 372VH-1 heavy chain variable region amino acid sequence (SEQ ID NO: 17): 
EVQLQESGGGLVKPGGSLKLSCAASGFTFSDYGMHWVRQAPEKGLEWVAYISSGRSTIYS
ADTVKGRFTISRDNAKNTLFLQMTSLRSEDTAMYYCARGGYYGNAMDYWGQGTSVTVS
S;
372VH-1 heavy chain variable region gene sequence (SEQ ID NO: 18): 
GAGGTGCAGCTGCAGGAGTCTGGGGGAGGCTTAGTGAAGCCTGGAGGGTCCCTGAAA
CTCTCCTGTGCAGCCTCTGGATTCACTTTCAGTGACTATGGAATGCACTGGGTTCGTCA
GGCTCCAGAGAAGGGGCTGGAGTGGGTTGCATACATTAGTAGTGGCAGAAGTACCATC
 
TACTCTGCAGACACAGTGAAGGGCCGATTCACCATCTCCAGAGACAATGCCAAGAAC
ACCCTGTTCCTGCAAATGACCAGTCTGAGGTCTGAGGACACGGCCATGTATTACTGTG
CAAGGGGAGGTTACTACGGTAATGCTATGGACTACTGGGGTCAAGGAACCTCAGTCAC
CGTCTCCTCA.
2) 372VH-6 heavy chain variable region amino acid sequence (SEQ ID NO: 19): 
EVKLQESGPELVKPGASVKMSCKASGYTFTDYYMHWVKQKPGKGLEWIGEIYPGSGNT
YYNEKFKGKATLTADTSSSTAYMQLSSLTSEDSAVYFCAGGGDYYDYDGTGYYAMDYW
GQGTSVTVSS;
372VH-6 heavy chain variable region gene sequence (SEQ ID NO: 20): 
GAGGTGAAGCTGCAGGAGTCTGGACCTGAGCTGGTAAAGCCTGGGGCTTCAGTGAAG
ATGTCCTGCAAGGCTTCTGGATACACATTCACTGACTACTACATGCACTGGGTGAAGCA
GAAGCCTGGGAAGGGCCTTGAGTGGATTGGAGAGATTTATCCTGGAAGTGGTAATACT
TACTACAATGAGAAGTTCAAGGGCAAGGCCACACTGACTGCAGACACATCCTCCAGC
ACAGCCTACATGCAGCTCAGCAGCCTGACATCTGAGGACTCTGCAGTCTATTTCTGTGC
GGGGGGGGGGGACTACTATGATTACGACGGGACGGGTTACTATGCTATGGACTACTGG
GGTCAAGGAACCTCAGTCACCGTCTCCTCA.
3) 372VL-1 light chain variable region amino acid sequence (SEQ ID NO: 21): 
QIVLTQSPAIMSASPGEKVTLTCSASSSVSSSYLYWYQQKPGSSPKLWIYSTSNLASGVPAR

FSGSGSGTSYSLTISSMEAEDAASYFCHQWSSYPPTFGGGTKLEIK;
372VL-1 light chain variable region gene sequence (SEQ ID NO: 22):

CAAATTGTTCTCACCCAGTCTCCAGCAATCATGTCTGCATCTCCTGGGGAGAAGGTCAC
CTTGACCTGCAGTGCCAGCTCAAGTGTAAGTTCCAGCTACTTGTACTGGTACCAGCAG
AAGCCAGGATCCTCCCCCAAACTCTGGATTTATAGCACATCCAACCTGGCTTCTGGAGT

CCCTGCTCGCTTCAGTGGCAGTGGGTCTGGGACCTCTTACTCTCTCACAATCAGCAGC
ATGGAGGCTGAAGATGCTGCCTCTTATTTCTGCCATCAGTGGAGTAGTTACCCACCCAC
GTTCGGAGGGGGGACCAAGCTGGAAATAAAA.

### ➢ Antibody 394

1) 394VH-1 heavy chain variable region amino acid sequence (SEQ ID NO: 23): 
EVQLQESGETVKISCKASGYIFTTSGMSWVIQAPGKGLKWMGLINTYSGVPTYADDFKGR

FAFSLETSASTAYLQINNLENEDTATYFCAIWSRAWFPYWGQGTLVTVSA;
394VH-1 heavy chain variable region gene sequence (SEQ ID NO: 24): 
GAGGTGCAGCTGCAGGAGTCTGGAGAGACAGTCAAGATCTCCTGCAAGGCTTCTGGG
TATATTTTCACAACCTCTGGAATGAGTTGGGTGATTCAGGCTCCAGGAAAGGGTTTAAA
GTGGATGGGCTTGATAAACACCTACTCTGGAGTGCCAACATATGCTGATGACTTCAAGG
GACGGTTTGCCTTCTCTTTGGAAACCTCTGCCAGCACTGCCTATTTGCAGATCAACAAC
CTCGAAAATGAAGACACGGCTACATATTTCTGTGCAATATGGTCCCGGGCCTGGTTTCC
TTACTGGGGCCAAGGGACTCTGGTCACTGTCTCTGCA.
2) 394VH-2 heavy chain variable region amino acid sequence (SEQ ID NO: 25): 
EVKLQESGAELMKPGASVKLSCKATGYTFTGYWIEWVKQRPGHGLEWIGEILPGSGSTN
YNEKFKGKATFTADTSSNTAYMQLSSLTTEDSAIYYCARAPLEGLRSGFAYWGQGTLVTV
SA;
394VH-2 heavy chain variable region gene sequence (SEQ ID NO: 26): 
GAGGTGAAGCTGCAGGAGTCTGGAGCTGAGCTGATGAAGCCTGGGGCCTCAGTGAAG

CTTTCCTGCAAGGCTACTGGCTACACATTCACTGGCTACTGGATAGAGTGGGTAAAGC

AGAGGCCTGGACATGGCCTTGAGTGGATTGGAGAGATTTTACCTGGAAGTGGTAGTAC

TAACTACAATGAGAAGTTCAAGGGCAAGGCCACATTCACTGCAGATACATCCTCCAAC
 
ACAGCCTACATGCAACTCAGCAGCCTGACAACTGAGGACTCTGCCATCTATTACTGTG
CAAGAGCCCCCTTGGAAGGGCTACGGAGCGGGTTTGCTTACTGGGGCCAAGGGACTC
TGGTCACTGTCTCTGCA.
3) 394VL-1 light chain variable region amino acid sequence (SEQ ID NO: 27): 
DIVLTQSPASLAVSLGQRATISCRASESVDSYGNSFMHWYQQKPGQPPKLLIYRASNLESGI

PARFSGSGSRTDFTLTINPVEADDVATYYCQQSNEDPRTFGGGTKLEIK;
394VL-1 light chain variable region gene sequence (SEQ ID NO: 28): 
GACATTGTGCTGACCCAATCTCCAGCTTCTTTGGCTGTGTCTCTAGGGCAGAGGGCCA
CCATATCCTGCAGAGCCAGTGAAAGTGTTGATAGTTATGGCAATAGTTTTATGCACTGG
TACCAGCAGAAACCAGGACAGCCACCCAAACTCCTCATCTATCGTGCATCCAACCTAG
AATCTGGGATCCCTGCCAGGTTCAGTGGCAGTGGGTCTAGGACAGACTTCACCCTCAC
CATTAATCCTGTGGAGGCTGATGATGTTGCAACCTATTACTGTCAGCAAAGTAATGAGG
ATCCTCGGACGTTCGGTGGAGGCACCAAGCTGGAAATCAAA.
4) 394VL-4 light chain variable region amino acid sequence (SEQ ID NO: 29): 
DIVMTQSPATLSVTPGDRVSLSCRASQSISDYLHWYQQKSHESPRLLIKYASQSISGIPSRFS

GSGSGSDFTLSINSVEPEDVGVYYCQNGHSFPYTFGGGTKLEIK;
394VL-4 light chain variable region gene sequence (SEQ ID NO: 30):

GACATTGTGATGACCCAGTCTCCAGCCACCCTGTCTGTGACTCCAGGAGATAGAGTCT
CTCTTTCCTGCAGGGCCAGCCAGAGTATTAGCGACTACTTACACTGGTATCAACAAAAA
TCACATGAGTCTCCAAGGCTTCTCATCAAATATGCTTCCCAATCCATCTCTGGGATCCCC
TCCAGGTTCAGTGGCAGTGGATCAGGGTCAGATTTCACTCTCAGTATCAACAGTGTGG
AACCTGAAGATGTTGGAGTGTATTACTGTCAAAATGGTCACAGCTTTCCGTACACGTTC
GGAGGGGGGACCAAGCTGGAAATAAAA.

### Example 4: Humanized Anti-CLDN18.2 Antibodies

The framework regions of the variable regions of a part of the antibodies described above were humanized, and the specific sequences were as follows:

### 1) H239H-2a

H239H-2a heavy chain variable region amino acid sequence (SEQ ID NO: 31): 
EVQLLESGGGLVQPGGSLRLSCAASGFTFSDYGMHWVRQAPGKGLEWVAYISRGRSTTY
STDTVKGRFTISRDSSKNTLYLQMNSLRAEDTAVYYCARGSYYGNAMDYWGQGTLVTVS
S;
H239H-2a heavy chain variable region gene sequence (SEQ ID NO: 32):

GAAGTGCAGCTGCTGGAATCAGGAGGAGGACTGGTGCAGCCAGGAGGATCTCTGAGA
CTGTCTTGCGCAGCTTCTGGATTTACCTTCTCCGATTACGGCATGCATTGGGTGAGACA
GGCTCCAGGAAAAGGACTCGAGTGGGTGGCTTATATCTCTAGAGGAAGATCTACCACC
TACTCTACCGATACCGTGAAGGGAAGATTCACCATCTCCAGAGACTCTTCCAAGAACA
CCCTGTACCTGCAGATGAACTCTCTGAGAGCAGAGGATACAGCAGTGTACTATTGCGCT
AGAGGCTCTTACTACGGCAACGCTATGGACTATTGGGGACAGGGAACACTGGTGACAG
TGTCTTCA.

### 2) H239H-2b

H239H-2b heavy chain variable region amino acid sequence (SEQ ID NO: 33): 
EVQLLESGGGLVQPGGSLRLSCAASGFTFSDYGMHWVRQAPGKGLEWVAYISRGRSTTY
STDTVKGRFTISRDNSKNTLYLQMNSLRAEDTAVYYCARGSYYGNAMDYWGQGTLVTV
SS;
H239H-2b heavy chain variable region gene sequence (SEQ ID NO: 34):

GAAGTGCAGCTGCTGGAATCAGGAGGAGGACTGGTGCAGCCAGGAGGATCTCTGAGA
CTGTCTTGCGCAGCTTCTGGATTTACCTTCTCCGATTACGGCATGCATTGGGTGAGACA
GGCTCCAGGAAAAGGACTCGAGTGGGTGGCTTATATCTCTAGAGGAAGATCTACCACC
TACTCTACCGATACCGTGAAGGGAAGATTCACCATCTCCAGAGACAACTCCAAGAACA
CCCTGTACCTGCAGATGAACTCTCTGAGAGCAGAGGATACAGCAGTGTACTATTGCGCT
AGAGGCTCTTACTACGGCAACGCTATGGACTATTGGGGACAGGGAACACTGGTGACAG
TGTCTTCA.

### 3) H239K-6a

H239K-6a light chain variable region amino acid sequence (SEQ ID NO: 35): 
DIVMTQSPDSLAVSLGERATINCKSSQSLLNSGNQRNYLTWYQQKPGQPPKLLIYWASTRE
SGVPDRFSGSGSGTDFTLTISSLQAEDVAVYYCQSAYSYPFTFGGGTKLEIK;
H239K-6a light chain variable region gene sequence (SEQ ID NO: 36): 
GACATCGTGATGACCCAGTCTCCAGATTCTCTGGCAGTGTCTCTGGGAGAAAGAGCTA
CAATCAACTGCAAGTCCTCTCAGTCTCTGCTGAACTCCGGCAACCAGAGAAACTACCT
GACTTGGTACCAGCAGAAACCAGGACAGCCTCCTAAGCTGCTGATCTATTGGGCTTCT
ACCAGGGAATCCGGAGTGCCAGATAGATTTTCCGGATCTGGATCCGGCACCGATTTTAC
ACTGACAATCTCTTCTCTGCAGGCAGAAGACGTGGCTGTGTATTATTGCCAGTCCGCCT
ACTCCTACCCTTTTACCTTTGGCGGAGGAACAAAGCTGGAGATCAAG.
4) human IgG1 heavy chain constant region sequence (SEQ ID NO: 37): 







 


;
human IgG1 heavy chain constant region gene sequence (SEQ ID NO: 38): 
GCTTCTACCAAGGGACCTTCAGTGTTTCCTCTGGCTCCTTCTTCTAAGTCTACATCCGG
AGGAACAGCCGCACTGGGTTGTCTGGTGAAAGACTACTTCCCCGAACCGGTGACGGT
GTCGTGGAACTCAGGCGCCCTGACCAGCGGCGTGCACACCTTCCCGGCTGTCCTACAG
TCCTCAGGACTCTACTCCCTCAGCAGCGTGGTGACCGTGCCCTCCAGCAGCTTGGGCA
CCCAGACCTACATCTGCAACGTGAATCACAAGCCCAGCAACACCAAGGTGGACAAGA
AAGTTGAGCCCAAATCTTGTGACAAAACTCACACATGCCCACCGTGCCCAGCACCTGA
ACTCCTGGGGGGACCGTCAGTCTTCCTCTTCCCCCCAAAACCCAAGGACACCCTCATG
ATCTCCCGGACCCCTGAGGTCACATGCGTGGTGGTGGACGTGAGCCACGAAGACCCT
GAGGTCAAGTTCAACTGGTACGTGGACGGCGTGGAGGTGCATAATGCCAAGACAAAG
CCGCGGGAGGAGCAGTACAACAGCACGTACCGTGTGGTCAGCGTCCTCACCGTCCTG
CACCAGGACTGGCTGAATGGCAAGGAGTACAAGTGCAAGGTCTCCAACAAAGCCCTC
CCAGCCCCCATCGAGAAAACCATCTCCAAAGCCAAAGGGCAGCCCCGAGAACCACAG
GTGTACACCCTGCCCCCATCCCGGGATGAGCTGACCAAGAACCAGGTCAGCCTGACCT
GCCTGGTCAAAGGCTTCTATCCCAGCGACATCGCCGTGGAGTGGGAGAGCAATGGGC
AGCCGGAGAACAACTACAAGACCACGCCTCCCGTGCTGGACTCCGACGGCTCCTTCTT
CCTCTACAGCAAGCTCACCGTGGACAAGAGCAGGTGGCAGCAGGGGAACGTCTTCTC
ATGCTCCGTGATGCATGAGGCTCTGCACAACCACTACACGCAGAAGAGCCTCTCCCTG
TCTCCGGGTAAA.
The human kappa light chain constant region sequence was as follows (SEQ ID NO: 39): 


;
the human kappa light chain constant region gene sequence was as follows (SEQ ID NO: 40):

CGTACGGTGGCTGCACCATCTGTCTTCATCTTCCCGCCATCTGATGAGCAGTTGAAATC
TGGAACTGCCTCTGTTGTGTGCCTGCTGAATAACTTCTATCCCAGAGAGGCCAAAGTA
CAGTGGAAGGTGGATAACGCCCTCCAATCGGGTAACTCCCAGGAGAGTGTCACAGAG
CAGGACAGCAAGGACAGCACCTACAGCCTCAGCAGCACCCTGACGCTGAGCAAAGC
AGACTACGAGAAACACAAAGTCTACGCCTGCGAAGTCACCCATCAGGGCCTGAGCTC
GCCCGTCACAAAGAGCTTCAACAGGGGAGAGTGT.

### Example 5: Preparation of Recombinant Chimeric Antibodies and Humanized Antibodies

Restriction enzyme digestion sites (light chain: 5' HindIII: AAGCTT (SEQ ID NO: 85), 3' BsiWI: CGTACG (SEQ ID NO: 86); heavy chain: 5' HindIII: AAGCTT (SEQ ID NO: 85), 3' AgeI: ACCGGT (SEQ ID NO: 87)) were designed at both ends of the gene sequences of the variable regions of the antibodies described above, and a ribosome binding site (kozak sequence: GCCGCCACC, SEQ ID NO: 88) and a signal peptide were added after the 5' HindIII enzyme digestion sites.

The light chain signal peptide (MDMRVLAQLLGLLLLCFPGARC (SEQ ID NO: 89), corresponds to the nucleotide sequence: ATGGACATGAGGGTGCTGGCCCAGCTGCTGGGACTGCTG CTGCTGTGCTTCCCAGGCGCCAGATGC (SEQ ID NO: 90));
the heavy chain signal peptide (MGWSLILLFLVAVATRVLS (SEQ ID NO: 91), corresponds t o the nucleotide sequence: ATGGGTTGGTCTCTGATTCTCCTGTTTCTGGTGGCAGTGGC TACAAGAGTCCTGTCA (SEQ ID NO: 92)).

The sequences described above were synthesized to obtain VH and VL gene fragments of the antibody. The IgG1 constant region gene sequence of the heavy chain was linked to an expression vector pcDNA3.1 by enzyme digestion (the enzyme digestion sites of the heavy chain constant region were AgeI and EcoRI), and the kappa constant region gene sequence of the light chain was linked to an expression vector pcDNA3.1 by enzyme digestion (the enzyme digestion sites of the light chain constant region were BsiwI and EcoRI); the synthesized VH gene fragment of the antibody was digested with HindIII&AgeI , the synthesized VL gene fragment of the antibody was digested with HindIII&BsiWI, and the VH gene fragment of the antibody and the VL gene fragment of the antibody were linked to the heavy chain expression vector described above that had been digested with HindIII&AgeI (to construct a heavy chain full-length expression plasmid) and the light chain expression vector described above that had been digested with HindIII&BsiWI (to construct a light chain full-length expression plasmid), respectively, so as to obtain heavy chain and light chain expression plasmids of a chimeric antibody beginning with "CH" and a humanized antibody beginning with "H".

The CDRs described above are summarized in Table 2, and the composition of the CDRs are shown in Table 3.

**Table 2. VH CDR1-3 (i.e., HCDR1-3) and VL CDR1-3 (i.e., LCDR1-3)**

| Amino acid sequence of VH CDR1 | Sequence No. | Amino acid sequence of VH CDR2 | Sequence No. | Amino acid sequence of VH CDR3 | Sequence No. |
|---|---|---|---|---|---|
| SYYIH | 41 | WIYPGSGNTKYNEKFKG | 42 | GMDYGNYYLDY | 43 |
| DYGMH | 44 | YISRGRSTTYSTDTVKG | 45 | GSYYGNAMDY | 46 |
| NTYIY | 47 | RIDPANGNTKYAPKFQG | 48 | SPAYYINYYAMDY | 49 |
| / | / | YISSGRSTIYSADTVKG | 50 | GGYYGNAMDY | 51 |
| DYYMH | 52 | EIYPGSGNTYYNEKFKG | 53 | GGDYYDYDGTGYYAMDY | 54 |
| TSGMS | 55 | LINTYSGVPTYADDFKG | 56 | WSRAWFPY | 57 |
| GYWIE | 58 | EILPGSGSTNYNEKFKG | 59 | APLEGLRSGFAY | 60 |

| Amino acid sequence of VL CDR1 | Sequence No. | Amino acid sequence of VL CDR2 | Sequence No. | Amino acid sequence of VL CDR3 | Sequence No. |
|---|---|---|---|---|---|
| KASQDVGTAVA | 61 | WASTRHT | 62 | QQYSSYLT | 63 |
| HASQNINVWLS | 64 | KASNLHT | 65 | QQGQSYPYT | 66 |
| SASSSVSYMH | 67 | DTSKLAS | 68 | QQWSSNPFT | 69 |
| KASQNVGTNVA | 70 | STSYRYS | 71 | QQYNSYPLT | 72 |
| KSSQSLLNSGNQRNYLT | 73 | WASTRES | 74 | QSAYSYPFT | 75 |
| SASSSVSSSYLY | 76 | STSNLAS | 77 | HQWSSYPPT | 78 |
| RASESVDSYGNSFMH | 79 | RASNLES | 80 | QQSNEDPRT | 81 |
| RASQSISDYLH | 82 | YASQSIS | 83 | QNGHSFPYT | 84 |

**Table 3. Composition of heavy chain and light chain CDRs of antibodies**

| Name | Sequence number of VH CDR1 | Sequence number of VH CDR2 | Sequence number of VH CDR3 |
|---|---|---|---|
| 101VH-1 | 41 | 42 | 43 |
| 239VH-2 | 44 | 45 | 46 |
| 239VH-9 | 47 | 48 | 49 |
| 372VH-1 | 44 | 50 | 51 |
| 372VH-6 | 52 | 53 | 54 |
| 394VH-1 | 55 | 56 | 57 |
| 394VH-2 | 58 | 59 | 60 |

| Name | Sequence number of VL CDR1 | Sequence number of VL CDR2 | Sequence number of VL CDR3 |
|---|---|---|---|
| 101VL-1 | 61 | 62 | 63 |
| 101VL-2 | 64 | 65 | 66 |
| 239VL-1 | 67 | 68 | 69 |
| 239VL-4 | 70 | 71 | 72 |
| 239VL-6 | 73 | 74 | 75 |
| 372VL-1 | 76 | 77 | 78 |
| 394VL-1 | 79 | 80 | 81 |
| 394VL-4 | 82 | 83 | 84 |

### Example 6: Expression and Purification of Recombinant Antibodies

The plasmids expressing the light and heavy chains of the antibody, respectively, were transiently transfected into HEK293F cells (ATCC) at a ratio of 1:1 using PEI reagent (Cat. No. 24765-1, Polysciences). Before the transfection, the cell viability was above 95%. The cell density was adjusted to 1.5-2 million cells/mL during the transfection. 1 µg of plasmid was used for every 1 million cells, and 3 µL of PEI was used for every 1 µg of plasmid. The desired plasmid and PEI were separately diluted with OPM-293 CD05 medium (Cat. No. 81075-001, OPM). The volume of the diluted plasmid or PEI was 2.5% of the total volume and the diluted plasmid and PEI were left to stand at room temperature for 5 min. The PEI was then added to the plasmid and mixed well. The mixture was left to stand at room temperature for 20 min. The plasmid-PEI complex was added to the cells and the cells were cultured on a shaker at 100 rpm. 24 h after the transfection, a feed OPM-CHO PFF05 (Cat. No. F81279-001; OPM) with a volume ratio of 10% was added to the cells. After 5-7 days, expression supernatants were collected, centrifuged at high speed to remove impurities, and purified using a Protein A column. The column was rinsed with PBS. The antibody protein was eluted with an acidic eluent of pH 3.0-3.5 and was adjusted to neutral with 1 M Tris-HCl. The elution sample was concentrated with an ultrafiltration tube, buffer-exchanged into a PBS buffer, aseptically filtered, and aliquoted for storage.

The chimeric and humanized antibodies prepared are shown in Table 4; the antibody beginning with "CH" denotes a chimeric antibody (murine variable region + human constant region) and the antibody beginning with "H" denotes a humanized antibody (humanized variable region + human constant region).

**Table 4. Composition of antibodies**

| Name of antibody | Sequence number of VH | Sequence number of CH | Sequence number of VL | Sequence number of CL |
|---|---|---|---|---|
| CH239H-2-K-6 | SEQ ID NO:7 | SEQ ID NO:37 | SEQ ID NO: 15 | SEQ ID NO:39 |
| CH239H-9-K-4 | SEQ ID NO:9 | SEQ ID NO:37 | SEQ ID NO:13 | SEQ ID NO:39 |
| CH394H2-K-1 | SEQ ID NO:25 | SEQ ID NO:37 | SEQ ID NO:27 | SEQ ID NO:39 |
| CH372H6-K-1 | SEQ ID NO:19 | SEQ ID NO:37 | SEQ ID NO:21 | SEQ ID NO:39 |
| CH239H-9-K-6 | SEQ ID NO:9 | SEQ ID NO:37 | SEQ ID NO:15 | SEQ ID NO:39 |
| CH239H-9-K-1 | SEQ ID NO:9 | SEQ ID NO:37 | SEQ ID NO:11 | SEQ ID NO:39 |
| CH101H1-K-1 | SEQ ID NO:1 | SEQ ID NO:37 | SEQ ID NO:3 | SEQ ID NO:39 |
| CH372H1-K-1 | SEQ ID NO:17 | SEQ ID NO:37 | SEQ ID NO:21 | SEQ ID NO:39 |
| H239H-2a-K-6a | SEQ ID NO:31 | SEQ ID NO:37 | SEQ ID NO:35 | SEQ ID NO:39 |
| H239H-2b-K-6a | SEQ ID NO:33 | SEQ ID NO:37 | SEQ ID NO:35 | SEQ ID NO:39 |

### Example 7: Binding of Anti-CLDN18.2 Antibodies to CLDN18.2 Expressed on Cell Surface

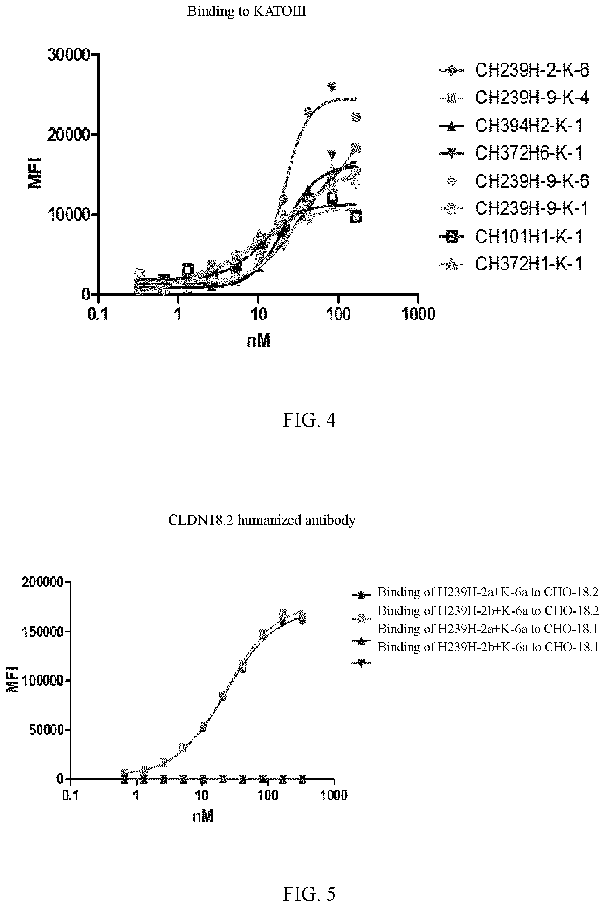

Flow cytometry was used to detect the binding capacity of anti-CLDN18.2 antibodies to cells expressing CLDN18.2, and the specificity of not binding to CLDN18.1 of the antibody was further detected. The experimental procedure overview was as follows: CLDN18.2 positive cells (KATOIII or CHO-CLDN18.2) and negative cells (CHO-CLDN18.1) were collected, washed once with PBS, resuspended in PBS, and plated on a 96-well V-bottom plate (Cat. No. 3897, Costar) at 0.1 million cells/50 µL/well. The antibody was pre-diluted to 333.2 nM with PBS, then diluted 2-fold with 9 gradients, added at 50 µL/well to the cells in the 96-well V-bottom plate described above, and mixed well (final antibody concentrations: 166.6, 83.3, 41.6, 20.8, 10.4, 5.2, 2.6, 1.3, 0.65, and 0.325 nM). The mixture was incubated at 4 °C for 1 h and washed twice with PBS. Then 100 µL of 1:500 diluted goat anti-human IgG-Fc PE fluorescent secondary antibody (Cat. No. 12-4998-82, eBioscience) was added. The mixture was incubated at 4 °C for 1 h, washed twice with PBS, and analyzed with a CytoFLEX flow cytometer (model: AOO-1-1102, Beckman).
1) The detection of the chimeric antibodies and the cells expressing CLDN18.2 is shown in FIG. 4. The chimeric antibodies CH239H-2-K-6, CH239H-9-K-4, CH394H2-K-1, CH372H6-K-1, CH239H-9-K-6, CH239H-9-K-1, CH101H1-K-1, and CH372H1-K-1 all could bind to CLDN18.2 on KATOIII cells, wherein the binding activity of the chimeric Antibody CH239H-2-K-6 was better.
2) The binding capacity of the chimeric Antibody CH239H-2-K-6 to CLDN18.2 on KATOIII cells was better than that of the positive Antibody IMAB362: the EC₅₀ values of IMAB362 and CH239H-2-K-6 were 8.082 nM and 6.290 nM, respectively.
3) After the chimeric Antibody CH239H-2-K-6 was humanized, the binding capacity of the humanized antibodies H239H-2a-K-6a and H239H-2b-K-6a to CLDN18.2 positive cells KATOIII and CHO-CLDN18.2 was substantially identical to that of the chimeric Antibody CH239H-2-K-6, and the affinity was not reduced; the Antibody CH239H-2-K-6, the Antibody H239H-2a-K-6a, and the Antibody H239H-2b-K-6a bound to KATOIII cells with EC₅₀ values of 6.108 nM, 6.203 nM, and 6.920 nM, respectively; the Antibody CH239H-2-K-6, the Antibody H239H-2a-K-6a, the Antibody H239H-2b-K-6a, and the positive Antibody IMAB362 bound to CHO-CLDN18.2 cells with EC₅₀ values of 19.92 nM, 22.83 nM, 23.31 nM, and 30.02 nM, respectively.
4) As shown in FIG. 5, the humanized Antibodies H239H-2a-K-6a and H239H-2b-K-6a bound only to CLDN18.2 and not to CLDN18.1.

### Example 8: ADCC (Antibody Dependent Cell-Mediated Cytotoxicity) Activity of Anti-CLDN18.2 Antibodies

ADCC Reporter Bioassay was selected in this experiment to evaluate ADCC activity of CLDN18.2 antibodies. This is a bioluminescent reporter gene assay system, which replaces NK cells using artificially constructed effector cells and quantifies the biological activity of the therapeutic antibody medicine based on the ADCC mechanism of action in the activation pathway with a high-sensitivity detection reagent, is a mechanism of action detection method. The cells expressing CLDN18.2 (CHO-CLDN18.2) were used as target cells and Jurkat-NFAT-luc-FcyRIIIa as effector cells (FcγRIIIa (sequence from NCBI, NP_001121065.1) was transfected into Jurkat (Cell Bank of Chinese Academy of Sciences, Shanghai, Clone E6-1) cells by lentivirus infection, and a positive clone was selected using 0.3 µg/mL puromycin (Cat. No. A11138-03, Gibco) and named Jurkat-FcyRIIIa; then the plasmid pGL4.30 [luc2P/NFAT-RE/Hygro] (Cat. No. E848A, Promega) containing a fluorescein reporter gene was transfected into the Jurkat-FcyRIIIa cells by electrotransfection, a positive clone was selected using hygromycin B (Cat. No. A600230-0001, Sangon Biotech), and the cells finally obtained were named Jurkat-NFAT-luc-FcγRIIIa), and the detection was performed using ONE-Glo^{™} Luciferase Assay System (Cat. No. E6120-100ml, Promega). The specific procedures were as follows: The CHO-CLDN18.2 cells were collected and washed once with RPMI1640 medium. Then the cell density was adjusted to 1.2 × 10⁶ cells/mL with RPMI1640 medium. The cells were inoculated in a 96-well white plate (Cat. No. 3917, Costar) at 25 µL/well, i.e., 30 thousand cells/well, and the plate was gently tapped to well mix the cells. The antibodies were pre-diluted to 10 µg/mL using an RPMI1640 medium containing 1% FBS as the antibody diluent, then diluted 5-fold with 8 gradients, and added to the cell plate described above at 50 µL/well. The plate was gently tapped to well mix the mixture. Then Jurkat-NFAT-luc-FcyRIIIa cells in the logarithmic phase were collected and washed once with RPMI1640 medium. Then the cell density was adjusted to 2.4 × 10⁶ cells/mL with RPMI1640 medium. The cells were added to the cell culture plate described above at 25 µL/well, i.e., 60 thousand cells/well, and mixed well. The culture plate was cultured in a 37 °C/5% CO₂ incubator for 6 h, and then taken out and placed at room temperature for 10 min to return to room temperature. 50 µL of ONE-GLO substrate (Cat. No. E6120-100ml, promega) was added. The culture plate was tested in a microplate reader (model: SpectraMax M3, Molecular Devices) to read the relative fluorescence unit (RLU).

CHO-CLDN18.1 cells were used as cell negative control, IMAB362 as antibody positive control, and the IgG isotype (Cat. No. BE0297, Bio cell) as antibody negative control.

The results show that the Antibody H239H-2b-k-6a had a strong ADCC effect, and the Antibody H239H-2b-k-6a had a stronger ADCC effect than the positive Antibody IMAB362: the EC₅₀ values corresponding to the Antibody IMAB362 and the Antibody H239H-2b-k-6a were 0.7953 nM and 0.06253 nM, respectively.

### Example 9: CDC (Complement Dependent Cytotoxicity) Activity of Anti-CLDN18.2 Antibodies

The CHO-CLDN18.2 cells were collected and washed once with PBS. The cell density was adjusted to 6.6 × 10⁵ cells/mL with RPMI1640 medium. The cells were plated on a 96-well flat-bottom plate at 45 µL/well. The antibodies were pre-diluted to 50 µg/mL using an RPMI1640 medium, and then diluted 4-fold with 9 gradients, and wells without added antibodies were used as control wells. Human serum complement (Cat. No. Quidel, A113) with a final concentration of 5% was used as a complement source, followed by incubation at 37 °C for 5 h. CCK-8 reagent (Cat. No. CK04, DojinDo) was added. The number of live cells was detected, and the cell killing rate [% cell killing rate = (1 - antibody well reading / control well reading) × 100] was calculated. The results show that the Antibody H239H-2b-k-6a had a significant CDC effect in the presence of complement, and the Antibody H239H-2b-k-6a had a stronger CDC effect than the positive Antibody IMAB362: the IC₅₀ values corresponding to the Antibody IMAB362 and the Antibody H239H-2b-k-6a were 5.867 nM and 0.2909 nM, respectively.

## Claims

1. An anti-CLDN18.2 antibody or an antigen-binding fragment thereof, the antibody or the antigen-binding fragment thereof comprising a heavy chain variable region and a light chain variable region,
wherein the heavy chain variable region comprises 1, 2, or 3 CDRs as follows:
a VH CDR1 comprising or consisting of an amino acid sequence: SYYIH (SEQ ID NO: 41),
DYGMH (SEQ ID NO: 44), NTYIY (SEQ ID NO: 47), DYYMH (SEQ ID NO: 52), TSGMS (SEQ ID NO: 55), or GYWIE (SEQ ID NO: 58);
a VH CDR2 comprising or consisting of an amino acid sequence: WIYPGSGNTKYNEKFKG (SEQ ID NO: 42), YISRGRSTTYSTDTVKG (SEQ ID NO: 45), RIDPANGNTKYAPKFQG (SEQ ID NO: 48), YISSGRSTIYSADTVKG (SEQ ID NO: 50), EIYPGSGNTYYNEKFKG (SEQ ID NO: 53), LINTYSGVPTYADDFKG (SEQ ID NO: 56), or EILPGSGSTNYNEKFKG (SEQ ID NO: 59);
a VH CDR3 comprising or consisting of an amino acid sequence: GMDYGNYYLDY (SEQ ID NO: 43), GSYYGNAMDY (SEQ ID NO: 46), SPAYYINYYAMDY (SEQ ID NO: 49), GGYYGNAMDY (SEQ ID NO: 51), GGDYYDYDGTGYYAMDY (SEQ ID NO: 54), WSRAWFPY (SEQ ID NO: 57), or APLEGLRSGFAY (SEQ ID NO: 60); and
the light chain variable region comprises 1, 2, or 3 CDRs as follows:
a VL CDR1 comprising or consisting of an amino acid sequence: KASQDVGTAVA (SEQ ID NO: 61), HASQNINVWLS (SEQ ID NO: 64), SASSSVSYMH (SEQ ID NO: 67), KASQNVGTNVA (SEQ ID NO: 70), KSSQSLLNSGNQRNYLT (SEQ ID NO: 73), SASSSVSSSYLY (SEQ ID NO: 76), RASESVDSYGNSFMH (SEQ ID NO: 79), or RASQSISDYLH (SEQ ID NO: 82);
a VL CDR2 comprising or consisting of an amino acid sequence: WASTRHT (SEQ ID NO: 62), KASNLHT (SEQ ID NO: 65), DTSKLAS (SEQ ID NO: 68), STSYRYS (SEQ ID NO: 71), WASTRES (SEQ ID NO: 74), STSNLAS (SEQ ID NO: 77), RASNLES (SEQ ID NO: 80), or YASQSIS (SEQ ID NO: 83);
a VL CDR3 comprising or consisting of an amino acid sequence: QQYSSYLT (SEQ ID NO: 63), QQGQSYPYT (SEQ ID NO: 66), QQWSSNPFT (SEQ ID NO: 69), QQYNSYPLT (SEQ ID NO: 72), QSAYSYPFT (SEQ ID NO: 75), HQWSSYPPT (SEQ ID NO: 78), QQSNEDPRT (SEQ ID NO: 81), or QNGHSFPYT (SEQ ID NO: 84).

2. The antibody or the antigen-binding fragment thereof according to claim 1, wherein the heavy chain variable region of the antibody or the antigen-binding fragment thereof comprises or consists of the following CDRs:
VH CDR1: SYYIH (SEQ ID NO: 41);
VH CDR2: WIYPGSGNTKYNEKFKG (SEQ ID NO: 42);
VH CDR3: GMDYGNYYLDY (SEQ ID NO: 43); or
VH CDR1: DYGMH (SEQ ID NO: 44);
VH CDR2: YISRGRSTTYSTDTVKG (SEQ ID NO: 45);
VH CDR3: GSYYGNAMDY (SEQ ID NO: 46); or
VH CDR1: NTYIY (SEQ ID NO: 47);
VH CDR2: RIDPANGNTKYAPKFQG (SEQ ID NO: 48);
VH CDR3: SPAYYINYYAMDY (SEQ ID NO: 49); or
VH CDR1: DYGMH (SEQ ID NO: 44);
VH CDR2: YISSGRSTIYSADTVKG (SEQ ID NO: 50);
VH CDR3: GGYYGNAMDY (SEQ ID NO: 51); or
VH CDR1: DYYMH (SEQ ID NO: 52);
VH CDR2: EIYPGSGNTYYNEKFKG (SEQ ID NO: 53);
VH CDR3: GGDYYDYDGTGYYAMDY (SEQ ID NO: 54); or
VH CDR1: TSGMS (SEQ ID NO: 55);
VH CDR2: LINTYSGVPTYADDFKG (SEQ ID NO: 56);
VH CDR3: WSRAWFPY (SEQ ID NO: 57); or
VH CDR1: GYWIE (SEQ ID NO: 58);
VH CDR2: EILPGSGSTNYNEKFKG (SEQ ID NO: 59);
VH CDR3: APLEGLRSGFAY (SEQ ID NO: 60); and
the light chain variable region of the antibody or the antigen-binding fragment thereof comprises or consists of the following CDRs:
VL CDR1: KASQDVGTAVA (SEQ ID NO: 61);
VL CDR2: WASTRHT (SEQ ID NO: 62);
VL CDR3: QQYSSYLT (SEQ ID NO: 63); or
VL CDR1: HASQNINVWLS (SEQ ID NO: 64);
VL CDR2: KASNLHT (SEQ ID NO: 65);
VL CDR3: QQGQSYPYT (SEQ ID NO: 66); or
VL CDR1: SASSSVSYMH (SEQ ID NO: 67);
VL CDR2: DTSKLAS (SEQ ID NO: 68);
VL CDR3: QQWSSNPFT (SEQ ID NO: 69); or
VL CDR1: KASQNVGTNVA (SEQ ID NO: 70);
VL CDR2: STSYRYS (SEQ ID NO: 71);
VL CDR3: QQYNSYPLT (SEQ ID NO: 72); or
VL CDR1: KSSQSLLNSGNQRNYLT (SEQ ID NO: 73);
VL CDR2: WASTRES (SEQ ID NO: 74);
VL CDR3: QSAYSYPFT (SEQ ID NO: 75); or
VL CDR1: SASSSVSSSYLY (SEQ ID NO: 76);
VL CDR2: STSNLAS (SEQ ID NO: 77);
VL CDR3: HQWSSYPPT (SEQ ID NO: 78); or
VL CDR1: RASESVDSYGNSFMH (SEQ ID NO: 79);
VL CDR2: RASNLES (SEQ ID NO: 80);
VL CDR3: QQSNEDPRT (SEQ ID NO: 81); or
VL CDR1: RASQSISDYLH (SEQ ID NO: 82);
VL CDR2: YASQSIS (SEQ ID NO: 83);
VL CDR3: QNGHSFPYT (SEQ ID NO: 84).

3. The antibody or the antigen-binding fragment thereof according to claim 1 or 2, wherein the antibody or the antigen-binding fragment thereof comprises a heavy chain variable region and a light chain variable region, wherein the heavy chain variable region comprises a VH CDR1 set forth in SEQ ID NO: 41, a VH CDR2 set forth in SEQ ID NO: 42, and a VH CDR3 set forth in SEQ ID NO: 43, and the light chain variable region comprises a VL CDR1 set forth in SEQ ID NO: 61, a VL CDR2 set forth in SEQ ID NO: 62, and a VL CDR3 set forth in SEQ ID NO: 63; or
the heavy chain variable region comprises a VH CDR1 set forth in SEQ ID NO: 41, a VH CDR2 set forth in SEQ ID NO: 42, and a VH CDR3 set forth in SEQ ID NO: 43, and the light chain variable region comprises a VL CDR1 set forth in SEQ ID NO: 64, a VL CDR2 set forth in SEQ ID NO: 65, and a VL CDR3 set forth in SEQ ID NO: 66; or
the heavy chain variable region comprises a VH CDR1 set forth in SEQ ID NO: 44, a VH CDR2 set forth in SEQ ID NO: 45, and a VH CDR3 set forth in SEQ ID NO: 46, and the light chain variable region comprises a VL CDR1 set forth in SEQ ID NO: 67, a VL CDR2 set forth in SEQ ID NO: 68, and a VL CDR3 set forth in SEQ ID NO: 69; or
the heavy chain variable region comprises a VH CDR1 set forth in SEQ ID NO: 44, a VH CDR2 set forth in SEQ ID NO: 45, and a VH CDR3 set forth in SEQ ID NO: 46, and the light chain variable region comprises a VL CDR1 set forth in SEQ ID NO: 70, a VL CDR2 set forth in SEQ ID NO: 71, and a VL CDR3 set forth in SEQ ID NO: 72; or
the heavy chain variable region comprises a VH CDR1 set forth in SEQ ID NO: 44, a VH CDR2 set forth in SEQ ID NO: 45, and a VH CDR3 set forth in SEQ ID NO: 46, and the light chain variable region comprises a VL CDR1 set forth in SEQ ID NO: 73, a VL CDR2 set forth in SEQ ID NO: 74, and a VL CDR3 set forth in SEQ ID NO: 75; or
the heavy chain variable region comprises or consists of a VH CDR1 set forth in SEQ ID NO: 47, a VH CDR2 set forth in SEQ ID NO: 48, and a VH CDR3 set forth in SEQ ID NO: 49, and the light chain variable region comprises or consists of a VL CDR1 set forth in SEQ ID NO: 67, a VL CDR2 set forth in SEQ ID NO: 68, and a VL CDR3 set forth in SEQ ID NO: 69; or
the heavy chain variable region comprises a VH CDR1 set forth in SEQ ID NO: 47, a VH CDR2 set forth in SEQ ID NO: 48, and a VH CDR3 set forth in SEQ ID NO: 49, and the light chain variable region comprises a VL CDR1 set forth in SEQ ID NO: 70, a VL CDR2 set forth in SEQ ID NO: 71, and a VL CDR3 set forth in SEQ ID NO: 72; or
the heavy chain variable region comprises a VH CDR1 set forth in SEQ ID NO: 47, a VH CDR2 set forth in SEQ ID NO: 48, and a VH CDR3 set forth in SEQ ID NO: 49, and the light chain variable region comprises a VL CDR1 set forth in SEQ ID NO: 73, a VL CDR2 set forth in SEQ ID NO: 74, and a VL CDR3 set forth in SEQ ID NO: 75; or
the heavy chain variable region comprises a VH CDR1 set forth in SEQ ID NO: 44, a VH CDR2 set forth in SEQ ID NO: 50, and a VH CDR3 set forth in SEQ ID NO: 51, and the light chain variable region comprises a VL CDR1 set forth in SEQ ID NO: 76, a VL CDR2 set forth in SEQ ID NO: 77, and a VL CDR3 set forth in SEQ ID NO: 78; or
the heavy chain variable region comprises a VH CDR1 set forth in SEQ ID NO: 52, a VH CDR2 set forth in SEQ ID NO: 53, and a VH CDR3 set forth in SEQ ID NO: 54, and the light chain variable region comprises a VL CDR1 set forth in SEQ ID NO: 76, a VL CDR2 set forth in SEQ ID NO: 77, and a VL CDR3 set forth in SEQ ID NO: 78; or
the heavy chain variable region comprises a VH CDR1 set forth in SEQ ID NO: 55, a VH CDR2 set forth in SEQ ID NO: 56, and a VH CDR3 set forth in SEQ ID NO: 57, and the light chain variable region comprises a VL CDR1 set forth in SEQ ID NO: 79, a VL CDR2 set forth in SEQ ID NO: 80, and a VL CDR3 set forth in SEQ ID NO: 81; or
the heavy chain variable region comprises a VH CDR1 set forth in SEQ ID NO: 55, a VH CDR2 set forth in SEQ ID NO: 56, and a VH CDR3 set forth in SEQ ID NO: 57, and the light chain variable region comprises a VL CDR1 set forth in SEQ ID NO: 82, a VL CDR2 set forth in SEQ ID NO: 83, and a VL CDR3 set forth in SEQ ID NO: 84; or
the heavy chain variable region comprises a VH CDR1 set forth in SEQ ID NO: 58, a VH CDR2 set forth in SEQ ID NO: 59, and a VH CDR3 set forth in SEQ ID NO: 60, and the light chain variable region comprises a VL CDR1 set forth in SEQ ID NO: 79, a VL CDR2 set forth in SEQ ID NO: 80, and a VL CDR3 set forth in SEQ ID NO: 81; or
the heavy chain variable region comprises a VH CDR1 set forth in SEQ ID NO: 58, a VH CDR2 set forth in SEQ ID NO: 59, and a VH CDR3 set forth in SEQ ID NO: 60, and the light chain variable region comprises a VL CDR1 set forth in SEQ ID NO: 82, a VL CDR2 set forth in SEQ ID NO: 83, and a VL CDR3 set forth in SEQ ID NO: 84.

4. The antibody or the antigen-binding fragment thereof according to claim 1, wherein the heavy chain variable region of the antibody or the antigen-binding fragment thereof comprises a sequence set forth in any one of SEQ ID NO: 1, SEQ ID NO: 7, SEQ ID NO: 9, SEQ ID NO: 17, SEQ ID NO: 19, SEQ ID NO: 23, and SEQ ID NO: 25, or a sequence having at least 90% identity to the sequences described above, or an amino acid sequence having one or more conservative amino acid substitutions as compared to the sequences described above; and/or the light chain variable region of the antibody or the antigen-binding fragment thereof comprises a sequence set forth in any one of SEQ ID NO: 3, SEQ ID NO: 5, SEQ ID NO: 11, SEQ ID NO: 13, SEQ ID NO: 15, SEQ ID NO: 21, SEQ ID NO: 27, and SEQ ID NO: 29, or a sequence having at least 90% identity to the sequences described above, or an amino acid sequence having one or more conservative amino acid substitutions as compared to the sequences described above.

5. An anti-CLDN18.2 antibody or an antigen-binding fragment thereof, wherein a heavy chain variable region of the antibody or the antigen-binding fragment thereof has an amino acid sequence set forth in SEQ ID NO: 1, and a light chain variable region has an amino acid sequence set forth in SEQ ID NO: 3 or SEQ ID NO: 5;
or the heavy chain variable region of the antibody or the antigen-binding fragment thereof has an amino acid sequence set forth in SEQ ID NO: 7 or SEQ ID NO: 9, and the light chain variable region has an amino acid sequence set forth in SEQ ID NO: 11, SEQ ID NO: 13, or SEQ ID NO: 15;
or the heavy chain variable region of the antibody or the antigen-binding fragment thereof has an amino acid sequence set forth in SEQ ID NO: 17 or SEQ ID NO: 19, and the light chain variable region has an amino acid sequence set forth in SEQ ID NO: 21;
or the heavy chain variable region of the antibody or the antigen-binding fragment thereof has an amino acid sequence set forth in SEQ ID NO: 23 or SEQ ID NO: 25, and the light chain variable region has an amino acid sequence set forth in SEQ ID NO: 27 or SEQ ID NO: 29.

6. The antibody or the antigen-binding fragment thereof according to any of claims 1-5, wherein the antibody or the antigen-binding fragment thereof is a humanized antibody.

7. An anti-CLDN18.2 antibody or an antigen-binding fragment thereof, wherein a heavy chain variable region of the antibody or the antigen-binding fragment thereof comprises a sequence set forth in SEQ ID NO: 31 or SEQ ID NO: 33, or a sequence having at least 90% identity to the sequences described above, or an amino acid sequence having one or more conservative amino acid substitutions as compared to the sequences described above; and/or
a light chain variable region of the antibody or the antigen-binding fragment comprises a sequence set forth in SEQ ID NO: 35, or a sequence having at least 90% identity to the sequence described above, or an amino acid sequence having one or more conservative amino acid substitutions as compared to the sequence described above.

8. The antibody or the antigen-binding fragment thereof according to any of claims 1-7, wherein the antibody is an IgG1 antibody.

9. An anti-CLDN18.2 antibody, wherein the antibody is CH239H-2-K-6, comprising a heavy chain variable region having an amino acid sequence set forth in SEQ ID NO: 7, a heavy chain constant region having an amino acid sequence set forth in SEQ ID NO: 37, a light chain variable region having an amino acid sequence set forth in SEQ ID NO: 15, and a light chain constant region having an amino acid sequence set forth in SEQ ID NO: 39;
or the antibody is CH239H-9-K-4, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 13, and a light chain constant region having an amino acid sequence set forth in SEQ ID NO: 39;
or the antibody is CH394H2-K-1, comprising a heavy chain variable region having an amino acid sequence set forth in SEQ ID NO: 25, a heavy chain constant region having an amino acid sequence set forth in SEQ ID NO: 37, a light chain variable region having an amino acid sequence set forth in SEQ ID NO: 27, and a light chain constant region having an amino acid sequence set forth in SEQ ID NO: 39;
or the antibody is CH372H6-K-1, comprising a heavy chain variable region having an amino acid sequence set forth in SEQ ID NO: 19, a heavy chain constant region having an amino acid sequence set forth in SEQ ID NO: 37, a light chain variable region having an amino acid sequence set forth in SEQ ID NO: 21, and a light chain constant region having an amino acid sequence set forth in SEQ ID NO: 39;
or the antibody is CH239H-9-K-6, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 15, and a light chain constant region having an amino acid sequence set forth in SEQ ID NO: 39;
or the antibody is CH239H-9-K-1, comprising a heavy chain variable region having an amino acid sequence set forth in SEQ ID NO: 9, a heavy chain constant region having an amino acid sequence set forth in SEQ ID NO: 37, a light chain variable region having an amino acid sequence set forth in SEQ ID NO: 11, and a light chain constant region having an amino acid sequence set forth in SEQ ID NO: 39;
or the antibody is CH101H1-K-1, comprising a heavy chain variable region having an amino acid sequence set forth in SEQ ID NO: 1, a heavy chain constant region having an amino acid sequence set forth in SEQ ID NO: 37, a light chain variable region having an amino acid sequence set forth in SEQ ID NO: 3, and a light chain constant region having an amino acid sequence set forth in SEQ ID NO: 39;
or the antibody is CH372H1-K-1, comprising a heavy chain variable region having an amino acid sequence set forth in SEQ ID NO: 17, a heavy chain constant region having an amino acid sequence set forth in SEQ ID NO: 37, a light chain variable region having an amino acid sequence set forth in SEQ ID NO: 21, and a light chain constant region having an amino acid sequence set forth in SEQ ID NO: 39;
or the antibody is H239H-2a-K-6a, comprising a heavy chain variable region having an amino acid sequence set forth in SEQ ID NO: 31, a heavy chain constant region having an amino acid sequence set forth in SEQ ID NO: 37, a light chain variable region having an amino acid sequence set forth in SEQ ID NO: 35, and a light chain constant region having an amino acid sequence set forth in SEQ ID NO: 39;
or the antibody is H239H-2b-K-6a comprising a heavy chain variable region having an amino acid sequence set forth in SEQ ID NO: 33, a heavy chain constant region having an amino acid sequence set forth in SEQ ID NO: 37, a light chain variable region having an amino acid sequence set forth in SEQ ID NO: 35, and a light chain constant region having an amino acid sequence set forth in SEQ ID NO: 39;
or the antibody comprises a sequence having at least 80% identity to any of the antibodies described above, or an amino acid sequence having one or more conservative amino acid substitutions as compared to any of the antibodies described above.

10. A nucleic acid molecule comprising a nucleotide encoding the antibody or the antigen-binding fragment thereof according to any of claims 1-9.

11. A nucleic acid molecule, wherein the nucleic acid molecule has a nucleotide sequence set forth in SEQ ID NO: 2, SEQ ID NO: 8, SEQ ID NO: 10, SEQ ID NO: 18, SEQ ID NO: 20, SEQ ID NO: 24, SEQ ID NO: 26, SEQ ID NO: 32, or SEQ ID NO: 34, or a sequence having at least 90% identity to the sequences described above.

12. A nucleic acid molecule, wherein the nucleic acid molecule has a nucleotide sequence set forth in SEQ ID NO: 4, SEQ ID NO: 6, SEQ ID NO: 12, SEQ ID NO: 14, SEQ ID NO: 16, SEQ ID NO: 22, SEQ ID NO: 28, SEQ ID NO: 30, or SEQ ID NO: 36, or a sequence having at least 90% identity to the sequences described above.

13. A biomaterial, being
(1) a vector, a host cell, or a microorganism, comprising the nucleic acid molecule according to any of claims 10-12; or
(2) an expression product, a suspension, or a supernatant, of the (1) described above.

14. The biomaterial according to claim 13, wherein the host cell is a CHO cell or an HEK293 cell.

15. A composition comprising the antibody or the antigen-binding fragment thereof according to any of claims 1-9.

16. The composition according to claim 15, wherein the composition is a pharmaceutical composition further comprising a pharmaceutically acceptable carrier.

17. A method for preparing the antibody or the antigen-binding fragment thereof according to any of claims 1-9, comprising: culturing the host cell according to claim 13 to express the antibody or the antigen-binding fragment and isolating the antibody or the antigen-binding fragment thereof.

18. The method according to claim 17, wherein the host cell is a CHO cell or an HEK293 cell.

19. Use of the antibody or the antigen-binding fragment thereof according to any of claims 1-9 or the nucleic acid molecule according to any of claims 10-12 or the biomaterial according to claim 13 or 14 or the composition according to claim 15 or 16 in the manufacture of a medicament for diagnosing or treating a cancer or a tumor.

20. The use according to claim 19, wherein the cancer or the tumor is a cancer or a tumor positive for CLDN18.2 expression.

21. The use according to claim 19, wherein the cancer or the tumor is selected from bladder cancer, ovarian cancer, lung cancer, adenocarcinoma, gastric cancer, breast cancer, liver cancer, pancreatic cancer, skin cancer, malignant melanoma, head and neck cancer, sarcoma, bile duct cancer, renal cancer, colon cancer, small intestine cancer, testicular embryonal carcinoma, placental choriocarcinoma, cervical cancer, testicular cancer, uterine cancer, esophageal cancer, and gallbladder cancer cells.
